# EUROPEAN PATENT APPLICATION

(11) **EP 3 757 208 A1**
(43) Date of publication of application: **30.12.2020**
(21) Application number: 19754255.8
(22) Date of filing: 18.02.2019
(51) Int. Cl.: C12N 5/0793, A61K 35/30, A61L 27/38, A61P 9/10, A61P 21/02, A61P 25/00, A61P 25/14, A61P 25/16, A61P 25/28, A61P 27/02, A61P 43/00, C12N 5/074

(54) **CELL AGGREGATE, MIXTURE OF CELL AGGREGATES, AND METHOD FOR PREPARING SAME**

(30) Priority: 19.02.2018 JP 2018027455
(71) Applicant: Sumitomo Dainippon Pharma Co., Ltd., Chuo-ku Osaka-shi Osaka 541-8524 (JP)
(72) Inventor: YOSHIDA Kenji, Kobe-shi, Hyogo 650-0047 (JP); YOSHIKAWA Manabu, Suita-shi, Osaka 564-0053 (JP); SEKIYA Sayaka, Kobe-shi, Hyogo 650-0047 (JP)
(74) Representative: Hoffmann Eitle
(86) International application number: PCT/JP2019/005914
(87) International publication number: WO 2019/160148

(57) **Abstract**

An object of the present invention is to provide a cell aggregate comprising dopaminergic neuron progenitor cells suitable for transplantation, a mixture of cell aggregates, and a method for producing these. The cell aggregate of the present invention comprises FOXA2-positive or TUJ1-positive neural cells and comprising 1000 cells or more.

## Description

### Technical Field

The present invention relates to an adherent cell population such as a cell aggregate, a mixture of the cell populations and a method for producing them.

### Background Art

Parkinson's disease is a neurodegenerative disease that is developed by loss of dopaminergic neural cells in the mesencephalic substantia nigra. At present, there are about four million patients with Parkinson's disease in the world. As treatments of Parkinson's disease, a drug treatment with L-DOPA or a dopamine agonist, coagulation with stereoencephalotomy, a deep brain stimulation therapy, transplantation of fetal mesencephalic cells, and the like are carried out. The transplantation of fetal mesencephalic cells has an ethical problem with its source of supply as well as a high risk of infection.

Recently, a therapy using dopaminergic neural cells or progenitor cells thereof, i.e., dopaminergic neuron progenitor cells prepared by induction from pluripotent stem cells such as embryonic stem cells (ES cells) and induced pluripotent stem cells (iPS cells) has been proposed (Non Patent Literature 1), and a method for producing the cells has been reported. More specifically, as a method for producing dopaminergic neuron progenitor cells, a method comprising selecting and separating cells suitable for transplantation with a factor (more specifically, Corin or Lrtm1) serving as a marker for dopaminergic neural cells or dopaminergic neuron progenitor cells is suggested (Patent Literature 1, Non Patent Literature 2 and Non Patent Literature 3). However, a further improvement has been desired in order to reduce the influence of difference between lots, thereby ensuring uniformity in quality and increase production efficiency.

### Citation List

### Patent Literature

Patent Literature 1: International Publication No. WO2015/34012

### Non Patent Literature

Non Patent Literature 1: Wernig M, et al., Proc Natl Acad Sci U SA. 2008, 105: 5856-5861
Non Patent Literature 2: Doi D, et al., Stem Cells Reports. 2014, 2: 337-350
Non Patent Literature 3: Samata B, et al., Nature communication. 2016, 7: 1-11

### Summary of Invention

### Technical Problem

An object of the present invention is to provide an adherent cell population such as a cell aggregate of neuronal cells having a satisfactory size and shape, a mixture of highly uniform cell aggregates or cell populations containing the adherent cell population, and a method for producing them, and more specifically a cell aggregate containing dopaminergic neuron progenitor cells, a mixture of highly uniform cell aggregates and a method for producing them.

### Solution to Problem

As a result of intensive studies, the present inventors found that a cell aggregate containing a suitable number of neural cells for human transplantation requiring proper control of cells in number and condition, and a homogeneous mixture of the cell aggregates can be obtained by: suspending the plurality of cells in a continuous flow of a liquid vehicle; selecting and separating the desired neuronal precursor cells through separating the cells into desired neuronal precursor cells and other cells so as to let them flow into different continuous flows of the liquid vehicle; and culturing the desired neuronal precursor cells to produce a cell aggregate containing neural cells. Based on the finding, the present invention was accomplished.

More specifically, the present invention relates to the following.
[1] A cell aggregate comprising FOXA2-positive or TUJ1-positive neural cells and comprising 1000 or more cells.
[2] The cell aggregate according to [1], comprising about 70% or more of the FOXA2-positive or TUJ1-positive neural cells, based on a total number of cells.
[3] The cell aggregate according to [1] or [2], wherein cell death can be suppressed during culture.
[4] The cell aggregate according to any of [1] to [3], further having at least one characteristic selected from the following:
   (a1) equivalent circle diameter is 100 µm to 2000 µm;
   (a2) convexity or solidity is 0.5 or more;
   (a3) Feret diameter ratio is 0.5 or more; and
   (a4) circularity is 0.3 or more.
[5] The cell aggregate according to any of [1] to [4], wherein the cell aggregate has no debris layer on a surface thereof, and a borderline of the cell aggregate is clear under a microscope.
[6] A mixture of a plurality of cell aggregates, comprising 50% or more of the cell aggregate according to any of [1] to [5], based on a total number of cell aggregates.
[7] The mixture of cell aggregates according to [6], wherein at least one index selected from the group consisting of a circularity, a minimum diameter, a maximum diameter, a vertical Feret diameter or a horizontal Feret diameter, a Feret diameter ratio, an equivalent circle diameter, a perimeter, an area, and a convexity or a solidity has a coefficient of variation of 15% or less.
[8] A method for producing a mixture of adherent cell populations, comprising steps of:
   (1) inducing differentiation of a plurality of stem cells in the presence of a first differentiation-inducing factor to obtain a plurality of cells comprising one or more neuronal precursor cells in a first differentiation stage;
   (2) selectively separating the neuronal precursor cells in a first differentiation stage from the plurality of cells obtained in step (1), wherein the separating step comprises
      suspending the plurality of cells obtained in step (1) in a continuous flow of a liquid vehicle, and
      distinguishing the neuronal precursor cells in a first differentiation stage, and separating the neuronal precursor cells in a first differentiation stage and other cells so as to let the neuronal precursor cells in a first differentiation stage and the other cells flow into different continuous flows of the liquid vehicle; and
   (3) culturing the neuronal precursor cells in a first differentiation stage, separated in step (2) in the presence of a second differentiation-inducing factor to obtain a mixture of adherent cell populations, wherein the mixture of adherent cell populations comprises 50% or more of adherent cell populations having the following characteristics (b1) and (b2), based on a total number of the adherent cell populations:
      (b1) comprising neural cells in a second differentiation stage; and
      (b2) comprising 1000 or more cells.
[9] The production method according to [8], wherein cell death of the adherent cell populations having characteristics (b1) and (b2) can be suppressed.
[10] The production method according to [9], wherein, when the adherent cell populations are cultured for 14 to 20 days, a number of cells at the completion of culture is 5% or more and preferably 10% or more of a number of cells at the beginning of culture.
[11] The production method according to any of [8] to [10], wherein the mixture of adherent cell populations is a mixture of cell aggregates.
[12] The production method according to [11], wherein the adherent cell populations are cell aggregates, and the above cell aggregates having characteristics (b1) and (b2) have an equivalent circle diameter of 100 µm to 2000 µm.
[13] The production method according to [12], wherein the adherent cell populations having characteristics (b1) and (b2) are cell aggregates, which further have the following characteristics:
   (b3) convexity or solidity is 0.5 or more;
   (b4) Feret diameter ratio is 0.5 or more; and
   (b5) circularity is 0.3 or more.
[14] The production method according to any of [11] to [13], wherein at least one index selected from the group consisting of a circularity, a minimum diameter, a maximum diameter, a vertical Feret diameter or a horizontal Feret diameter, a Feret diameter ratio, an equivalent circle diameter, a perimeter, an area and, a convexity or a solidity of the mixture of cell aggregates has a coefficient of variation of 15% or less.
[15] The production method according to any of [8] to [14], wherein, in step (2), the neuronal precursor cells in a first differentiation stage are separated by using a micro-channel system cell sorter.
[16] The production method according to any of [8] to [15], wherein, in step (2), the neuronal precursor cells in a first differentiation stage are separated in a closed system.
[17] The production method according to any of [8] to [16], wherein the stem cells are pluripotent stem cells.
[18] The production method according to any of [8] to [17], wherein the neuronal precursor cells in a first differentiation stage are neuronal precursor cells committed to midbrain floor plate.
[19] The production method according to [18], wherein the neuronal precursor cells in a first differentiation stage are Corin-positive and/or Lrtm1-positive cells.
[20] The production method according to any of [8] to [19], wherein the neural cells in a second differentiation stage are neural cells positive for at least one marker selected from the group consisting of TUJ1, OTX2, FOXA2, LMX1A, LMX1B, EN1, Nurr1, PITX3, DAT, GIRK2 and TH.
[21] The production method according to [20], wherein the neural cells in a second differentiation stage are FOXA2-positive and TUJ1-positive dopaminergic neuron progenitor cells.
[22] A mixture of adherent cell populations obtained by the production method according to any of [8] to [21].
[23] A method for producing an adherent cell population, comprising separating the adherent cell populations having characteristics (b1) and (b2) from the mixture of adherent cell populations obtained by the production method according to any of [8] to [21].
[24] An adherent cell population obtained by the production method according to [23].
[25] A pharmaceutical composition for transplantation, comprising any of the cell aggregate according to any of [1] to [5]; the mixture of cell aggregates according to [6] or [7]; the mixture of adherent cell populations according to [22]; and the adherent cell population according to [24].
[26] A therapeutic agent for a disease in need of supplement of neural cells, comprising any of the cell aggregate according to any of [1] to [5]; the mixture of cell aggregates according to [6] or [7]; the mixture of adherent cell populations according to [22]; and the adherent cell population according to [24].
[27] A method for treating a disease in need of supplement of neural cells, comprising transplanting any of the cell aggregate according to any of [1] to [5]; the mixture of cell aggregates according to [6] or [7]; the mixture of adherent cell populations according to [22]; and the adherent cell population according to [24], into a central nerve of a patient.

### Advantageous Effects of Invention

According to the present invention, it is possible to provide an adherent cell population such as a cell aggregate of neuronal cells having a satisfactory size and shape, a mixture of highly uniform adherent cell populations containing the above cell population, and a method for producing them. According to the present invention, it is possible to attain uniformity of adherent cell populations such as cell aggregates at a level required for a pharmaceutical product, and to provide neural cells suitable for transplantation to, for example, humans.

### Brief Description of Drawings

Figure 1 shows a protocol for induction of differentiation of human iPS cells into dopaminergic neuron progenitor cells.
Figure 2 shows microscopic images (n = 3) of cell aggregates in the second differentiation stage on 16th, 20th, 24th and 28th days (day 16, day 20, day 24, day 28) in suspension culture with respect to each of cell groups sorted by Jazz or Gigasort.
Figure 3 shows images for morphological observation of cell aggregates on 28th day (day 28) after initiation of differentiation induction observed by a digital microscope. (A) shows the results by Jazz; whereas (B) shows the results by Gigasort.
Figure 4 shows graphs showing the measurement results of equivalent circle diameter (A), convexity or solidity (B), area (C), Feret diameter ratio (D) and circularity (E) of cell aggregates of Figure 3, in each of which the case of Jazz (light gray) is compared to the case of Gigasort (dark gray).
Figure 5 shows a graph showing coefficients of variations (CV value) of a minimum diameter, a perimeter, a Feret diameter (horizontal), a Feret diameter (vertical), a Feret diameter ratio, a solidity, a convexity, an area, a maximum diameter, a circularity and an equivalent circle diameter of cell aggregates shown in Figure 3, calculated from the measurement results of cell aggregates. For each of the parameters, the CV value in the case of Jazz (light gray) is compared to that of Gigasort (dark gray).
Figure 6 shows images of cells obtained by immunostaining with an anti-FOXA2 antibody, an anti-Nurrl antibody, an anti-TH antibody and DAPI, on the 28th day (day 28) after initiation of differentiation induction.

### Description of Embodiments

### I. Definition

### <Cell population>

In the present specification, an adherent cell population refers to an aggregate of cells formed of a plurality of cells mutually adhered, and conceptually includes a three-dimensional adherent cell population, in which cells are three-dimensionally and biologically bound (namely, adhered), and a two-dimensional adherent cell population, in which cells are two-dimensionally and biologically bound.

The three-dimensional adherent cell population, which is also referred to as a cell aggregate, is not particularly limited as long as it is an aggregate of cells forming a three-dimensional structure and may be spherical or non-spherical. In the present specification, a cell aggregate is a cell aggregate preferably having a three-dimensional shape close to a sphere. The three-dimensional shape close to a sphere is a shape having a three-dimensional structure, whose figure projected onto a two-dimensional surface is, for example, a circle or ellipse.

The two-dimensional adherent cell population, which is also referred to as a cell sheet, is not particularly limited as long as it is a single-layered or multiple-layered construct formed by two-dimensional binding of single layered or multiple layered cells. A cell-sheet produced by adherent culture and a cell-sheet produced by non-adherent culture are both included in the cell sheet of the specification.

In the present specification, a "mixture of adherent cell populations" or a "mixture of cell aggregates" refers to an embodiment (composition) where two or more adherent cell populations or cell aggregates are present. The adherent cell populations or cell aggregates may be suspended in a liquid vehicle such as culture medium in a container, adhering to a container, or precipitated on the bottom of a container. A frozen adherent cell population or cell aggregate is also included in the mixture of adherent cell populations or cell aggregates in the present specification.

In the present specification, cells (including cells of a cell aggregate, a cell sheet, a cell population, or the like) refer to mammalian cells, preferably cells of a rodent (e.g., a mouse or a rat) or a primate (e.g., a human or a monkey), and more preferably, human cells.

### <Neural cells>

In the present specification, neural cells include all neural cells such as neural cells of the central nervous system; or neural cells of the peripheral nervous system such as neural cells of the autonomic nerve system or neural cells of the motor nerve system or the sensory system. Examples of the neural cells include neuronal cells, neural crest-derived cells, glial cells, oligodendrocytes, microglial cells, and stem cells or precursor cells thereof.

In the present specification, FOXA2-positive or TUJ1-positive neural cells are not particularly limited as long as they are neural cells expressing FOXA2 or TUJ1 at a detectable level. Examples of the neural cells include neural stem cells, neuronal precursor cells, neuronal cells, ventral midbrain-derived neuronal precursor cells, dopaminergic neuron progenitor cells, dopaminergic neural cells, GABA neuronal precursor cells, GABA neuronal cells, cholinergic neuronal precursor cells, cholinergic neuronal cells, glutamatergic neuronal precursor cells, glutamatergic neuronal cells, retinal cells (including, photoreceptor cells, photoreceptor precursor cells, retinal pigment epithelium cells, or the like) and corneal cells.

More specifically, examples of the FOXA2-positive and TUJ1-negative neural cells include neural stem cells, neuronal precursor cells and ventral midbrain-derived neuronal precursor cells.

Examples of the FOXA2-negative and TUJ1-positive neural cells include GABA neuronal precursor cells, GABA neuronal cells, cholinergic neuronal precursor cells, cholinergic neuronal cells, glutamatergic neuronal precursor cells, glutamatergic neuronal cells, retinal cells (including photoreceptor cells, photoreceptor precursor cells, and retinal pigment epithelium cells) and corneal cells.

Examples of the FOXA2-positive and TUJ1-positive neural cells include neuronal cells such as dopaminergic neuron progenitor cells and dopaminergic neural cells.

In the present specification, dopaminergic neuron progenitor cells may include dopaminergic neural cells or dopaminergic neurons, unless otherwise specified. The dopaminergic neuron progenitor cells are positive for FOXA2 and TUJ1, and further preferably include cells positive for one or more of OTX2, LMX1A, LMX1B, EN1, Nurr1, PITX3, DAT, GIRK2 and TH.

Another embodiment of the neural cells include neural cells positive for at least one of FOXA2, TUJ1, OTX2, LMX1A, LMX1B, EN1, Nurr1, PITX3, DAT, GIRK2 and TH.

Examples of human FOXA2 include a polynucleotide represented by NCBI accession number NM 021784 or NM_153675, and proteins encoded by these.

Examples of human TUJ1 (neuron-specific class III beta-tubulin) include a polynucleotide represented by NCBI accession number NM_006086 or NM 001197118, and proteins encoded by these.

Examples of human OTX2 include a polynucleotide represented by NCBI accession number NM_021728, NM_172337, NM_001270523, NM_001270524 or NM_001270525, and proteins encoded by these.

Examples of human LMX1A include a polynucleotide represented by NCBI accession number NM 001174069 or NM_177398, and proteins encoded by these.

Examples of human LMX1B include a polynucleotide represented by NCBI accession number NM 002316, NM 001174146 or NM_001174147, and proteins encoded by these.

Examples of human EN1 include a polynucleotide represented by NCBI accession number NM_001426, and a protein encoded by this.

Examples of human Nurr1 include a polynucleotide represented by NCBI accession number NM_006186, and a protein encoded by this.

Examples of human PITX3 include a polynucleotide represented by NCBI accession number NM_005029, and a protein encoded by this.

Examples of human DAT (SLC6A3) include a polynucleotide represented by NCBI accession number NM 001044, and a protein encoded by this.

Examples of human GIRK2 (KCNJ6) include a polynucleotide represented by NCBI accession number NM_002240, and a protein encoded by this.

Examples of human TH include a polynucleotide represented by NCBI accession number NM_000360, NM_199292 or NM_199293, and proteins encoded by these.

### <Neuronal precursor cells>

The neuronal precursor cells refer to precursor cells that can be further differentiated into neural cells. The neuronal precursor cells can be differentiated into any types of neural cells including neuronal cells, such as neural cells of the central nervous system; or neural cells of the peripheral nervous system such as neural cells of the autonomic nerve system or neural cells of the motor nerves system or the sensory system.

### <Stem cells>

In the present specification, stem cells refer to cells having both pluripotency (ability to differentiate into a plurality of types of cells) and replication competence that are capable of proliferating without limit. Examples of the stem cells include pluripotent stem cells such as embryonic stem cells (ES cells) and induced pluripotent stem cells (iPS cells) artificially prepared from cells derived from bone marrow, blood, or skin (epidermis, dermis, or subcutaneous tissue) by gene introduction; and somatic stem cells present in adipose, hair follicles, brain, nerves, liver, pancreas, kidneys, muscles, and other tissues that differentiate into a plurality of predetermined types of cells.

### <Pluripotent stem cells>

In the present specification, pluripotent stem cells are not particularly limited as long as they are stem cells having both pluripotency to differentiate into all types of cells present in a living body and proliferation potency.

The pluripotent stem cells can be induced from a fertilized egg, a cloned embryo, reproductive stem cells, tissue stem cells, somatic cells, or the like. Examples of the pluripotent stem cells include embryonic stem cells (ES cells), embryonic germ cells (EG cells) and induced pluripotent stem cells (iPS cells). Multi-lineage differentiating stress enduring cells (Muse cells) obtained from mesenchymal stem cells (MSC) and sperm stem cells produced from germ cells (for example, testis) (GS cells) are also included in the pluripotent stem cells. The embryonic stem cells were established for the first time in 1981, and have been used for producing knockout mice on and after 1989. In 1998, human embryonic stem cells were established, and it has come to be used in regenerative medicine. The embryonic stem cells may be produced by culturing an embryoblast on feeder cells or in a medium containing a leukemia inhibitory factor (LIF). Methods for producing embryonic stem cells is described, for example, in WO96/22362, WO02/101057, US5,843,780, US6,200,806 and US6,280,718. The embryonic stem cells are available from predetermined institutions, and are also commercially available. For example, human embryonic stem cells KhES-1, KhES-2 and KhES-3 are available from Kyoto University's Institute for Frontier Medical Sciences. Human embryonic stem cells Rx::GFP line (derived from KhES-1 line) are available from RIKEN, National Research and Development Institute. EB5 cell line and D3 cell line, which are mouse embryonic stem cells, are available from RIKEN, National Research and Development Institute, and ATCC, respectively.

Nuclear transfer embryonic stem cells (ntES cells), which are one of the embryonic stem cells, can be established from a cloned embryo prepared by transplanting the nucleus of a somatic cell into an egg from which a nucleus has been removed.

EG cells can be produced by culturing primordial germ cells in a medium containing mSCF, LIF and bFGF (Cells, 70: 841-847, 1992).

In the present specification, "induced pluripotent stem cells" refer to cells obtained by reprogramming a somatic cell in accordance with a known method to induce pluripotency. More specifically, examples of induced pluripotent stem cells include cells obtained by reprogramming a differentiated somatic cell, such as a fibroblast or a peripheral blood mononuclear cell, by expressing any of combinations of a plurality of genes selected from a group of reprogramming genes including Oct3/4, Sox2, Klf4, Myc (c-Myc, N-Myc, L-Myc), Glis1, Nanog, Sall4, Lin28, Esrrb, and the like. Preferable combinations of reprogramming factors include (1) Oct3/4, Sox2, Klf4 and Myc (c-Myc or L-Myc), and (2) Oct3/4, Sox2, Klf4, Lin28 and L-Myc (Stem Cells, 2013; 31: 458-466).

Induced pluripotent stem cells were established in mouse cells by Yamanaka, et al. in 2006 (Cells, 2006, 126 (4), pp. 663-676). Induced pluripotent stem cells were established also in human fibroblasts in 2007, and were found to have pluripotency and replication competence as with embryonic stem cells (Cells, 2007, 131 (5), pp. 861-872; Science, 2007, 318 (5858), pp. 1917-1920; Nat. Biotechnol., 2008, 26 (1), pp. 101-106).

Induced pluripotent stem cells may be produced not only by a direct reprogramming with a gene expression but also by a method inducing induced pluripotent stem cells from a somatic cell by addition of chemical compounds (Science, 2013, 341, pp. 651-654) or the like.

Induced pluripotent stem cells established as cell lines are also available, and for example, human induced pluripotent stem cell lines such as 201B7 cells, 201B7-Ff cells, 253G1 cells, 253G4 cells, 1201C1 cells, 1205D1 cells, 1210B2 cells and 1231A3 cells established in Kyoto University are available from Kyoto University. Induced pluripotent stem cell lines, for example, Ff-I01 cells, Ff-I01s04 cells, QHJ-I01 and Ff-I14 cells, established by Kyoto University, are available from Kyoto University.

Examples of somatic cells used for producing induced pluripotent stem cells include, but are not particularly limited to, tissue-derived fibroblasts, blood cells (for example, peripheral blood mononuclear cells (PBMC) or T cells), hepatocytes, pancreatic cells, intestinal epithelial cells and smooth muscle cells.

When induced pluripotent stem cells are produced by reprogramming by expressing several types of genes, the means for expressing the genes is not particularly limited. Examples of the means include an infection method using a virus vector (for example, retro-virus vector, lentivirus vector, Sendai virus vector, adenovirus vector or adeno-associated virus vector); a gene introduction method (for example, calcium phosphate method, lipofection method, RetroNectin method or electroporation method) using a plasmid vector (for example, plasmid vector or episomal vector); a gene introduction method (for example, calcium phosphate method, lipofection method or electroporation method) using an RNA vector; and a method (for example, method using a needle, lipofection method, or electroporation method) of directly injecting a protein.

Induced pluripotent stem cells may be produced in the presence of feeder cells or in the absence of feeder cells (feeder free). When induced pluripotent stem cells are produced in the presence of feeder cells, induced pluripotent stem cells may be produced by a known method, in the presence of a undifferentiation-maintaining factor. The culture medium used for producing induced pluripotent stem cells in the absence of feeder cells is not particularly limited, and a known maintenance medium for embryonic stem cells and/or induced pluripotent stem cells or a culture medium for establishing induced pluripotent stem cells in feeder-free conditions may be used. Examples of the culture medium for establishing induced pluripotent stem cells in feeder-free conditions include feeder-free mediums such as Essential 8 medium (E8 medium), Essential 6 medium, TeSR medium, mTeSR medium, mTeSR-E8 medium, stabilized Essential 8 medium and StemFit medium. An induced pluripotent stem cell may be produced, for example, by introducing 4 factors, i.e., Oct3/4, Sox2, Klf4 and Myc genes, into a somatic cell in feeder-free conditions, by use of a Sendai virus vector.

The pluripotent stem cells used in the present invention are mammalian pluripotent stem cells, preferably pluripotent stem cells of a rodent (e.g., a mouse or a rat) or a primate (e.g., a human or a monkey), more preferably human or mouse pluripotent stem cells, and further preferably human induced pluripotent stem cells (iPS cells) or human embryonic stem cells (ES cells).

### <Differentiation-inducing factor>

A differentiation-inducing factor refers to a factor regulating intracellular signaling for inducing differentiation of stem cells to neural cells (including neuronal precursor cells in the first differentiation stage and neural cells in the second differentiation stage). Differentiation-inducing factors well known to those skilled in the art may be appropriately selected depending on the type of neural cell.

Examples of a differentiation-inducing factor used for inducing differentiation of pluripotent stem cells into Corin-and/or Lrtml-positive cells include a BMP inhibitor, a TGF-β inhibitor, a SHH signal stimulant, FGF8 and a GSK-3β inhibitor.

Examples of a differentiation-inducing factor used for inducing differentiation of Corin-positive and/or Lrtml-positive cells to dopaminergic neuron progenitor cells include a neurotrophic factor.

### <BMP inhibitor>

In the present specification, a BMP inhibitor is not particularly limited as long as it is a substance that inhibits signal transduction from BMP, and it may be any of a nucleic acid, a protein and a low molecular organic compound. Examples of the BMP include BMP2, BMP4, BMP7 and GDF7. Examples of the BMP inhibitor include substances that directly act on BMP (for example, an antibody or an aptamer); substances that inhibit expression of a gene encoding a BMP (for example, an antisense oligonucleotide or siRNA); substances that inhibit binding between a BMP receptor (BMPR) and a BMP; and substances that inhibit physiological activity caused by signal transduction through a BMP receptor. Examples of the BMPR include ALK2 and ALK3. As the BMP signal transduction pathway inhibiting substance, compounds well known to those skilled in the art can be used. Examples of the compounds include proteinaceous inhibitors such as Chordin, Noggin, Follistatin, Dorsomorphin (more specifically, 6-[4-(2-piperidin-1-yl-ethoxy)phenyl]-3-pyridin-4-yl-pyrazolo[1,5-a]pyr imidine) and derivatives thereof (P. B. Yu, et al. (2007), Circulation, 116: II_60; P.B. Yu, et al. (2008), Nat. Chem. Biol., 4: 33-41; J. Hao, et al. (2008), PLoS ONE, 3 (8): e2904), and LDN193189 (more specifically, 4-(6-(4-(piperazin-1-yl)phenyl)pyrazolo[1,5-a]pyrimidin-3-yl)quinoline ). LDN193189 herein is well known as a BMPR (ALK2/3) inhibitor (hereinafter referred to as a BMPR inhibitor) and is commercially available, for example, in a form of hydrochloride. Dorsomorphin and LDN193189 are available from Sigma-Aldrich and Stemgent, respectively. As the BMP inhibitor, one or two or more may be appropriately selected from these and put in use. The BMP inhibitor used in the present invention may be preferably LDN193189.

### <TGF-β inhibitor>

In the present specification, TGF-β inhibitor refers to a substance that inhibits binding of TGF-β to a TGF-β receptor followed by signal transduction to SMAD. The TGF-β inhibitor is not particularly limited as long as it inhibits a signal transduction pathway in which TGF-β is involved, and may be a nucleic acid, a protein or a low molecular organic compound. Examples of the substance include substances that directly act on TGF-β (for example, a protein, an antibody, or an aptamer); substances that inhibit the expression of a gene encoding TGF-β (for example, an antisense oligonucleotide or siRNA); substances that inhibit the binding between a TGF β receptor and TGF-β; and substances that inhibit physiological activity caused by a signal transduction through a TGF-β receptor (for example, a TGF β receptor inhibitor or an Smad inhibitor). TGF-β inhibitors may be a substance that inhibits binding to an ALK family serving as a receptor or a substance that inhibits phosphorylation of SMAD by an ALK family, and examples thereof include Lefty-1 (for example, mouse Lefty-1 represented by NCBI accession number NM_010094, and human Lefty-1 represented by NM 020997), Lefty-2 (for example, mouse Lefty-2 represented by NCBI accession number NM_177099, and human Lefty-2 represented by each of NM_003240 and NM 001172425), SB431542, SB202190 (both, see, R. K. Lindemann, et al., Mol. Cancer, 2003, 2: 20), SB505124 (GlaxoSmithKline), NPC30345, SD093, SD908, SD208 (Scios), LY2109761, LY364947, LY580276 (Lilly Research Laboratories), A83-01 (WO2009/146408), and derivatives thereof. The TGF-β inhibitor used in the present invention is preferably SB431542 (4-(5-benzol[1,3]dioxol-5-yl-4-pyridin-2-yl-1H-imidazol-2-yl)-benzami de) or A-83-01 (3-(6-methyl-2-pyridinyl)-N-phenyl-4-(4-quinolinyl)-1H-pyrazole-1-car bothioamide). These are known as inhibitors of a TGF-β receptor (ALK5) and an Activin receptor (ALK4/7). One or two or more may be appropriately selected from these and be used as a TGF-β inhibitor. TGF-β inhibitor used in the present invention may be further preferably A83-01.

Note that, the SMAD signal transduction inhibitory activity of a TGF-β inhibitor, a BMP inhibitor, or the like may be determined by a method well known to those skilled in the art, for example, by detecting the phosphorylation of Smad by western blotting method (Mol Cancer Ther. (2004) 3, 737-45.).

### <SHH signal stimulant>

In the present specification, a SHH (Sonic hedgehog) signal stimulant is defined as a substance that causes de-suppression of Smoothened (Smo), which is caused by binding of SHH to a receptor Patched (Ptch1), followed by activation of Gli2. Examples of the SHH signal stimulant include proteins belonging to the Hedgehog family, more specifically, SHH or IHH (Indian Hedgehog), a SHH receptor, a SHH receptor agonist, Hh-Ag1.5 (Li, X., et al., Nature Biotechnology, 23, 215 to 221 (2005)), a Smoothened Agonist, SAG (N-methyl-N'-(3-pyridinylbenzyl)-N'-(3-chlorobenzo[b]thiophene-2-car bonyl)-1,4-diaminocyclohexane), 20a-hydroxycholesterol, Purmorphamine (PMA: 9-cyclohexyl-N-[4-(4-morpholinyl)phenyl]-2-(1-naphthalenyloxy)-9H-p urin-6-amine), and derivatives thereof (Stanton BZ, Peng LF., Mol Biosyst. 6: 44-54, 2010). One or two or more may be appropriately selected from these and used as an SHH signal stimulant.

The SHH signal stimulant used in the present invention is preferably SHH protein (Genbank accession number: NM_000193, NP_000184), Purmorphamine, or SAG. The SHH signal stimulant used in the present invention may be further preferably Purmorphamine.

### <FGF8>

In the present specification, examples of FGF8 include, but are not particularly limited to, 4 splicing forms, FGF8a, FGF8b, FGF8e or FGF8f, and more preferably, FGF8 is FGF8b. FGF8 is commercially available from companies such as Wako and R&D systems and can be readily used. Alternatively, FGF8 may be obtained by forcibly expressing it in cells in accordance with a method known to those skilled in the art.

### <GSK-3β inhibitor>

In the present specification, GSK-3β inhibitor is defined as a substance that inhibits the kinase activity (for example, an ability to phosphorylate β-catenin) of GSK-3β protein. Although many substances are already known, examples thereof include an indirubin derivative BIO (also referred to as a GSK-3β inhibitor IX; 6-bromoindirubin 3'-oxime), a maleimide derivative SB216763 (3-(2,4-dichlorophenyl)-4-(1-methyl-1H-indol-3-yl)-1H-pyrrol-2,5-dion ), GSK-3β inhibitor VII (4-dibromoacetophenone), which is a phenyl α-bromomethyl ketone compound, a cell membrane permeable phosphorylated peptide L803-mts (also referred to as a GSK-3β peptide inhibitor: Myr-N-GKEAPPAPPQpSP-NH₂ (SEQ ID No. 1)), and highly selective CHIR99021 (6-[2-[4-(2,4-dichlorophenyl)-5-(4-methyl-1H-imidazol-2-yl)pyrimidin-2-ylamino]ethylamino]pyridine-3-carbonitrile). One or two or more may be appropriately selected and be used as a GSK-3β inhibitor. These compounds are commercially available, for example, from companies such as Calbiochem and Biomol and can be readily used. Alternatively, these compounds may be obtained from other supply sources or may be prepared by the user. The GSK-3β inhibitor used in the present invention may be preferably CHIR99021.

### <Extracellular matrix>

In the present specification, an extracellular matrix (also referred to as an extracellular substratum) refers to a supramolecular structure present outside a cell, and it may be naturally derived or artificially prepared (recombinant). Examples thereof include substances such as collagen, proteoglycan, fibronectin, hyaluronic acid, tenascin, entactin, elastin, fibrillin, and laminin, or fragments of these. These extracellular matrixes may be used in combination or prepared from cells, such as BD Matrigel (trademark). Preferably, the extracellular matrix is laminin or a fragment thereof. In the present specification, laminin is a protein having a heterotrimer structure having each one of a α chain, a β chain and a γ chain, and is an extracellular matrix protein which has isoforms having different compositions of subunit chains. Laminin is a heterotrimer of a combination of 5 types of α chains, 4 types of β chains and 3 types of γ chains, and has about 15 types of isoforms. Although not particularly limited, examples of the α chain include α1, α2, α3, α4 or α5; examples of the β chain include β1, β2, β3 or β4 and examples of the γ chain include γ1, γ2 or γ3. Laminin used in the present invention is more preferably laminin 511 consisting of a5, β1 and γ1 (Nat Biotechnol 28, 611-615 (2010)).

In the present invention, laminin may be a fragment, and the fragment is not particularly limited as long as it has an integrin binding activity. The fragment may, for example, be an E8 fragment obtained by digestion with elastase (EMBO J., 3: 1463-1468, 1984, J. Cells Biol., 105: 589-598, 1987) may be used. Accordingly, in the present invention, laminin 511E8 (preferably human laminin 511E8) described in WO2011/043405, which is obtained by digesting laminin 511 with elastase, is preferable. Note that, laminin E8 fragment such as laminin 511E8 used in the present invention needs not be a digestion product of laminin with elastase, and it may be a recombinant. Laminin 511E8 is also commercially available and can be purchased from, for example, Nippi Inc.

In order to avoid contamination with unidentified components, laminin or a laminin fragment used in the present invention is preferably isolated.

### <Neurotrophic factor>

In the present specification, a neurotrophic factor refers to a ligand to a membrane receptor and plays an important role in keeping motor neurons alive while maintaining function thereof. Examples thereof include a nerve growth factor (NGF), a brain-derived neurotrophic factor (BDNF), Neurotrophin 3 (NT-3), Neurotrophin 4/5 (NT-4/5), Neurotrophin 6 (NT-6), basic fibroblast growth factor (basic FGF), acidic fibroblast growth factor (acidic FGF), fibroblast growth factor-5 (FGF-5), epidermal growth factor (EGF), hepatocyte growth factor (HGF), insulin-like growth factor 1 (IGF-1), insulin-like growth factor 2 (IGF-2), glia cell line-derived neurotrophic factor (GDNF), TGF-β2, TGF-β3, interleukin-6 (IL-6), ciliary neurotrophic factor (CNTF) and LIF. One or two or more may be appropriately selected from these and put in use. A preferable neurotrophic factor in the present invention is a factor selected from the group consisting of GDNF and BDNF. A neurotrophic factor is commercially available from companies such as Wako and R&D systems and can be readily used. Alternatively, a neurotrophic factor may be obtained by forcibly expressing it in cells in accordance with a method known to those skilled in the art.

### <ROCK inhibitor>

In the present invention, a ROCK inhibitor is not particularly limited as long as it can suppress the function of Rho kinase (ROCK). Examples thereof include Y-27632 (see, for example, Ishizaki et al., Mol. Pharmacol. 57, 976-983 (2000), Narumiya et al., Methods Enzymol. 325,273-284 (2000)), Fasudil/HA1077 (see, for example, Uenata et al., Nature 389: 990-994 (1997)), H-1152 (see, for example, Sasaki et al., Pharmacol. Ther. 93: 225-232 (2002)), Wf-536 (see, for example, Nakajima et al., Cancer Chemother Pharmacol. 52 (4): 319-324 (2003)), and derivatives thereof; as well as an antisense nucleic acid to ROCK, an RNA interference-inducing nucleic acid (for example, siRNA), a dominant negative mutant, and expression vectors thereof. Other low molecular compounds are also known as a ROCK inhibitor, and such low molecular compounds or derivatives thereof may be used in the present invention (see, for example, U.S. Patent Application Nos. 20050209261, 20050192304, 20040014755, 20040002508, 20040002507, 20030125344 and 20030087919, and International Publication Nos. WO2003/062227, 2003/059913, 2003/062225, 2002/076976 and 2004/039796). In the present invention, one or two or more ROCK inhibitors may be used. The ROCK inhibitor used in the present invention may be preferably Y-27632.

### <Culture medium>

In the present specification, a culture medium used for culture of cells may be prepared from a culture medium routinely used for culturing animal cells as a basal medium. Examples of the basal medium include mediums that can be used for culturing animal cells, such as BME medium, BGJb medium, CMRL 1066 medium, Glasgow's Minimal Essential Medium (GMEM) medium, Improved MEM Zinc Option medium, IMDM medium, Medium 199 medium, Eagle MEM medium, aMEM medium, DMEM medium, F-12 medium, DMEM/F12 medium, StemFit medium, IMDM/F12 medium, Ham's medium, RPMI 1640 medium, Fischer's medium and Neurobasal medium, or a mixture of these mediums. From these basal mediums, the mediums used in individual steps of the production method of the present invention may be prepared.

In the present specification, a culture medium used for culturing a cell population containing pluripotent stem cells is desirably a medium containing an undifferentiation-maintaining factor (undifferentiation-maintaining medium), in order to inhibit cell death of the pluripotent stem cells. The culture medium used for culturing a cell population containing pluripotent stem cells is desirably a feeder-free and serum-free medium. The culture medium may be prepared, for example, by adding an undifferentiation-maintaining factor, a serum substitute and appropriate nutrition sources to a basal medium. More specifically, the culture medium may be prepared by adding bFGF, KSR, nonessential amino acids (NEAA), L-glutamine and 2-mercaptoethanol to DMEM/F12 medium.

In the present specification, "serum-free medium" refers to a culture medium not containing unadjusted or unpurified serum. In the present invention, a culture medium contaminated with a purified component derived from blood or a purified component derived from an animal tissue (for example, growth factor) is included in the serum-free medium, as long as it does not contain unadjusted or unpurified serum.

The serum-free medium may contain a serum substitute. The serum substitute may be albumin, transferrin, a fatty acid, a collagen precursor, trace elements, 2-mercaptoethanol or 3' thiol glycerol, or products containing equivalents of these as appropriate. The serum substitute may be prepared, for example, in accordance with a method described in WO98/30679. A commercially available serum substitute may also be used. Examples of the commercially available serum substitute include KnockOut Serum Replacement (KSR) manufactured by Life Technologies (current name: Thermo Fisher), Chemically-defined Lipid concentrated, Glutamax, B-27 Supplement, N2 Supplement and ITS Supplement.

The serum-free medium may contain a fatty acid or a lipid, an amino acid (for example, nonessential amino acid), a vitamin, a growth factor, a cytokine, an antioxidant, 2-mercaptoethanol, pyruvate, a buffer, an inorganic salt, or the like, as appropriate.

To avoid complexity in preparation, a serum-free medium prepared by adding an appropriate amount (for example, about 0.5% to about 30%, preferably about 1% to about 20%) of commercially available KSR (for example, a culture medium prepared by adding about 8% KSR and a chemically-defined lipid concentrated to GMEM medium) or a serum-free medium prepared by adding an appropriate amount (for example, about 0.1 to 5%) of commercially available B-27 to a neurobasal culture medium, may be used as the serum-free medium. As an equivalent to KSR, a culture medium disclosed in Japanese Unexamined Patent Publication No. 2001-508302 may be used.

Culture is preferably carried out in a serum-free medium. The serum-free medium is preferably a serum-free medium containing KSR or B-27, or a xeno-free medium. The "xeno-free" herein refers to conditions in which components derived from a species different from the species of cells to be cultured are eliminated.

In the present specification, feeder cells refer to cells that are allowed to be co-present with stem cells when the stem cells are cultured. Examples of the feeder cells include mouse fibroblasts (MEF or the like), human fibroblast, SNL cells and STO cells. The feeder cells may be feeder cells to which a growth suppression treatment is previously applied. The growth suppression treatment may be a treatment with a growth inhibitor (for example, mitomycin C) or a treatment with gamma irradiation, UV irradiation, or the like. However, in the present invention, culture is preferably carried out in the absence of feeder cells (feeder free).

In the present specification, "in the absence of feeder cells (feeder free)" refers to culture performed in the absence of feeder cells. The "feeder free" condition refers to a condition in which the feeder cells as mentioned above are not added or a condition substantially not containing feeder cells (for example, the ratio of feeder cells to a total number of cells is 3% or less, preferably 0.5% or less).

As the feeder-free medium that can be used as an undifferentiation-maintaining medium, many synthetic mediums have been developed and sold, such as Essential 8 medium. Essential 8 medium is DMEM/F12 medium containing L-ascorbic acid-2-phosphate magnesium (64 mg/L), sodium selenium (14 µg/l), insulin (19.4 mg/L), NaHCO₃ (543 mg/L), transferrin (10.7 mg/L), bFGF (100 ng/mL) and a TGF-β inhibitor (TGF-β1 (2 ng/mL) or Nodal (100 ng/mL)) as additives (Nature Methods, 8, 424-429 (2011)). Examples of a commercially available feeder-free medium include Essential 8 (manufactured by Life Technologies; current name: Thermo Fisher), S-medium (manufactured by DS PHARMA BIOMEDICAL CO., LTD.), StemPro (manufactured by Life Technologies; current name: Thermo Fisher), hESF9 (Proc Natl Acad Sci U S A. Sep 9, 2008; 105 (36): 13409-14), mTeSR1 (manufactured by STEMCELLS Technologies), mTeSR2 (manufactured by STEMCELLS Technologies company) and TeSR-E8 (manufactured by STEMCELLS Technologies). Other than these, feeder-free medium may be StemFit (manufactured by Ajinomoto Co., Inc.). By using these in step (1) above, the present invention can be carried out simply.

Note that, in the present specification, a "medium containing substance X" or "in the presence of substance X" refers to a medium to which an exogenous substance X is added or a medium containing an exogenous substance X; or in the presence of an exogenous substance X. More specifically, when a cell or a tissue present in the medium endogenously expresses, secretes or produces substance X, endogenous substance X is distinguished from an exogenous substance X, and the culture medium containing no exogenous substance X is interpreted as not falling within the scope of the "medium containing substance X", even if the medium contains endogenous substance X.

### II. Cell aggregate and mixture thereof

One embodiment of the present invention is a cell aggregate containing FOXA2-positive or TUJ1-positive neural cells, wherein the number of cells per aggregate is 1000 or more. A mixture of cell aggregates is a mixture of a plurality of cell aggregates, containing 50% or more of the cell aggregate of the present invention, based on the total number of cell aggregates.

In the cell aggregate, the number of FOXA2-positive neural cells or TUJ1-positive neural cells is not particularly limited as long as the cell aggregate or the cell aggregate-derived material can exert the function of neural cells upon transplantation into a living body, and it varies depending on the type of neural cells. The number of FOXA2-positive neural cells or TUJ1-positive neural cells is preferably about 70% or more, further preferably about 80% or more, and more preferably about 90% or more of the total number of cells.

One embodiment of the present invention is a cell aggregate containing FOXA2-positive and TUJ1-positive neuronal cells, wherein the number of cells per aggregate is 1000 or more.

When the neural cells are dopaminergic neuron progenitor cells, the cell aggregate of the present invention contains preferably about 50% or more, further preferably about 70% or more, and more preferably about 80% or more of FOXA2-positive and TUJ1-positive neuronal cells, based on the total number of cells.

In an embodiment of the present invention, the cell aggregate is characterized in that cell death can be suppressed during culture. The phrase "cell death can be suppressed during culture" means that cell death of neuronal cells, which usually occurs when cells are cultured in the presence of a differentiation-inducing factor or the like at 37°C, can be suppressed.

For example, when a cell aggregate is cultured at 37°C in the presence of a differentiation-inducing factor for 14 to 20 days, it can be determined that "cell death can be suppressed during culture" of the cell aggregate if the number of cells at the completion of culture is 5% or more, preferably 8% or more, further preferably 10% or more, further preferably 15% or more, and further preferably 30% or more of the number cells at the beginning of the culture.

In an embodiment of the present invention, the cell aggregate has at least one characteristic selected from the following (a1) to (a4). The cell aggregate may have all characteristics (a1) to (a4).
(a1) equivalent circle diameter is 100 µm to 2000 µm;
(a2) convexity or solidity is 0.5 or more;
(a3) Feret diameter ratio is 0.5 or more; and
(a4) circularity is 0.3 or more.

Herein, characteristics (a1) to (a4) may be measured by parallelly applying transillumination to a cell aggregate in a perpendicular direction to the observation surface of a microscope or a digital microscope, photographing the resultant image of the cell aggregate by a camera, and analyzing the figure (namely, a projected figure of the cell aggregate onto a flat plane).

The equivalent circle diameter herein refers to the diameter of a circle having the same area as that of the projected figure. The equivalent circle diameter is preferably 100 µm to 1000 µm, further preferably 200 µm to 600 µm, preferably 300 µm to 600 µm and further more preferably 450 µm to 600 µm.

The convexity or solidity represents the ratio of the perimeter or area of the projected figure and a convex polygon enveloping the figure. More specifically, there exists convexity (perimeter) and solidity (area), and the convexity refers to the ratio of the perimeter of a figure to the perimeter of a figure enveloping the figure, and the solidity refers to the ratio of the area of a figure to the area of a figure enveloping the figure. The solidity or convexity is preferably 0.7 to 1.0, further preferably 0.8 to 1.0.

The Feret diameter ratio refers to the ratio of the horizontal length and the vertical length orthogonal thereto of a tetragon circumscribing the above figure, and it is represented by the ratio of the vertical length to the horizontal length. The Feret diameter ratio is preferably 0.6 to 1.0, and further preferably 0.7 to 1.0.

The circularity is a value represented by the expression: 4π × (area) ÷ (perimeter)². When the above figure is a true circle, the circularity is 1. As the figure becomes elongated, the circularity gets closer to 0. The circularity is preferably 0.5 to 1.0, and further preferably 0.7 to 1.0.

One embodiment of the cell aggregate of the present invention is a cell aggregate having no debris layer formed on the surface of the isolated cell aggregate, and the borderline of the cell aggregate is clear under a microscope.

The microscope used herein is not particularly limited as long as it is a microscope of about 4 to 10 times magnification well known to those skilled in the art, and specifically, Thermo Fisher EVOS XL may be used.

The "isolated cell aggregate" refers to a cell aggregate that is not in contact with other cell aggregates, so that the outer edge thereof is observable.

The debris layer refers to a structure present on the surface of a cell aggregate, in which a group of particles (for example, dead cells), each of which can be observed as a single particle, is assembled to form a continuous layer. When the debris layer is formed on the surface of a cell aggregate, the borderline of the cell aggregate is unclear compared to that of a cell aggregate having no debris layer or having a little debris layer.

A mixture of cell aggregates containing a plurality of cell aggregates of the present invention falls within the scope of the present invention. In the present specification, a mixture of cell aggregates contains at least 2 or more, and preferably 5 or more cell aggregates, and contains about 20% or more, preferably about 40% or more, further preferably about 50% or more, and particularly preferably 60% or more of the cell aggregate of the present invention, based on the total number of cell aggregates. The mixture of cell aggregates may contain a small (but of measurable size) group of cells present in a satellite manner.

The "small group of cells present in a satellite manner" refers to a small group of cells that is present independently of the cell aggregates without binding to them, and that consists of a plurality of cells (for example, dead cells).

The mixture of cell aggregates of the present invention is satisfactorily uniform at least in size and shape, and at least one index selected from the group consisting of a circularity, a minimum diameter, perimeter, Feret diameter (vertical Feret diameter or horizontal Feret diameter), a Feret diameter ratio, a maximum diameter, a convexity or a solidity, an area, and an equivalent circle diameter has a coefficient of variation (CV value) of 15% or less, preferably 12% or less or 10% or less, and more preferably 8% or less or 5% or less. Individual indexes herein may be measured by parallelly applying transillumination to a cell aggregate in a perpendicular direction to the observation surface of a microscope or a digital microscope, photographing the resultant image of the cell aggregate by a camera, and analyzing the figure obtained. The measurement method is not limited as long as measurement can be made with almost the same accuracy as in this method.

The minimum diameter herein refers to a minimum value of the distance between two parallel lines when the figure is sandwiched by the two parallel lines. The minimum diameter of the cell aggregate of the present invention is, for example, 200 µm to 600 µm, preferably 300 µm to 600 µm, and further preferably 400 µm to 600 µm.

The perimeter is the length of periphery of a figure, and more specifically, refers to the length of periphery of a projected figure obtained by projecting a cell aggregate to a flat plane. The perimeter of the cell aggregate of the present invention is, for example, 800 µm to 2700 µm and preferably 1600 µm to 2700 µm.

The Feret diameter (vertical Feret diameter or horizontal Feret diameter) refers to the length in the vertical direction or the horizontal direction of a tetragon circumscribed to the figure. More specifically, in a case which a figure obtained by projecting a cell aggregate to a flat plate is assumed to be circumscribed by a tetragon, the lengths of individual sides of the tetragon are referred to as the Feret diameter. The vertical Feret diameter or horizontal Feret diameter of the cell aggregate of the present invention is, for example, 200 µm to 800 µm, preferably 300 µm to 600 µm and further preferably 400 µm to 800 µm.

The maximum diameter refers to a value showing the longest one of the distances between two points arbitrarily selected on the inner circumference of the figure. More specifically, the maximum diameter refers to a value showing the longest one of the distances between two points arbitrarily selected on the inner circumference of a figure, which is formed by projecting a cell aggregate to a flat plane. The maximum diameter of the cell aggregate of the present invention is, for example, 200 µm to 900 µm, preferably 300 µm to 600 µm, and further preferably 400 µm to 900 µm.

The area refers to the area of a figure calculated two dimensionally, and more specifically, refers to the area of a figure formed by projecting a cell aggregate to a flat plane. The area of the cell aggregate of the present invention is, for example, 46000 µm² to 278000 µm², and preferably 165000 µm² to 278000 µm².

Although the indexes mentioned above each have a plurality of values corresponding to the directions along which a cell aggregate is projected to a flat plane, a measured value along any direction may be employed for the sake of convenience. Among the indexes, the values of Feret diameter ratio, convexity or solidity, and circularity become more uniform as the shape of a cell aggregate comes closer to a true sphere, in other words, as the shape of a figure of a cell aggregate projected to a flat plane comes closer to a true circle.

### III. Method for producing mixture of adherent cell populations

One embodiment of the present invention is a method for producing a mixture of adherent cell populations containing neural cells, comprising steps of:
(1) inducing differentiation of a plurality of stem cells in the presence of a first differentiation-inducing factor to obtain a plurality of cells containing one or more neuronal precursor cells in a first differentiation stage;
(2) selectively separating a neuronal precursor cells in the first differentiation stage from the plurality of cells obtained in step (1), the step comprising suspending the plurality of cells obtained in step (1) in a continuous flow of a liquid vehicle, distinguishing the neuronal precursor cells in a first differentiation stage, and separating the neuronal precursor cells in a first differentiation stage and other cells so as to let the neuronal precursor cells in a first differentiation stage and the other cells flow into different continuous flows of the liquid vehicle; and
(3) culturing the neuronal precursor cells in a first differentiation stage, separated in step (2) in the presence of a second differentiation-inducing factor to obtain a mixture of adherent cell populations, wherein the mixture of adherent cell populations comprises 50% or more of adherent cell populations having the following characteristics (b1) and (b2), based on a total number of the adherent cell populations:
   (b1) containing neural cells in a second differentiation stage; and
   (b2) containing 1000 or more cells.

### <Step (1)>

Step (1) is a step of inducing differentiation of a plurality of stem cells in the presence of a first differentiation-inducing factor to obtain a plurality of cells containing one or more neuronal precursor cells in the first differentiation stage. In the present specification, neuronal precursor cells in the first differentiation stage are not particularly limited as long as they are neuronal precursor cells corresponding to intermediate cells obtained upon inducing differentiation of stem cells, preferably pluripotent stem cells, to neural cells in the second differentiation stage. The neuronal precursor cells in the first differentiation stage may, for example, be neuronal precursor cells that can differentiate into neuronal cells.

Specifically, the neuronal precursor cells may be neuronal precursor cells committed to the midbrain floor plate. The neuronal precursor cells committed to the midbrain floor plate may be Corin-positive and/or Lrtml-positive cells. The Corin-positive and/or Lrtml-positive cells can be produced by a method well known to those skilled in the art.

As a method of inducing differentiation of stem cells into neuronal precursor cells in the first differentiation stage, a method known to those skilled in the art may be used as appropriate, depending on the type of neuronal precursor cells. More specifically, culture may be carried out in an appropriate culture medium in the presence of a first differentiation-inducing factor well known to those skilled in the art. The first differentiation-inducing factor herein refers to a factor influencing the differentiation state (expression of transcription factors, genes, or proteins involved in differentiation) of cells, and examples thereof include a low molecular compound, a protein, a peptide fragment of a protein, and a physical factor such as carbon dioxide gas, oxygen partial pressure or pressure. More specifically, a method using an SMAD inhibitor (BMP inhibitor or TGF-β inhibitor), an SHH signal stimulant, a GSK-3β inhibitor, a neurotrophic factor, or the like is known.

For example, in the case of the neuronal precursor cells committed to the midbrain floor plate, a known method described in Stem cells reports, vol. 2 337-350, 2014 may be used.

In the present specification, specifically, the neuronal precursor cells committed to the midbrain floor plate may be Corin-positive and/or Lrtm1 -positive cells. The Corin-positive and/or Lrtm1-positive cells refer to cells in which Corin protein and/or Lrtm1 protein is expressed in a sufficient amount to be recognized by an anti-Corin antibody or an anti-Lrtml antibody.

A method for inducing differentiation of stem cells will be more specifically described by way of the case where the neuronal precursor cells in the first differentiation stage are neuronal precursor cells including Corin-positive and/or Lrtml-positive cells.

Induction of differentiation of pluripotent stem cells into Corin-positive and/or Lrtml-positive cells may be carried out in a medium containing a first differentiation-inducing factor. Examples of the first differentiation-inducing factor include a BMP inhibitor, a TGF-β inhibitor, an SHH signal stimulant, FGF8 and a GSK-3β inhibitor described above. Induction of differentiation of pluripotent stem cells into Corin-positive and/or Lrtml-positive cells is desirably carried out by the following steps:
(1a) subjecting pluripotent stem cells to adherent culture performed on an extracellular matrix (also referred to as an extracellular substratum) in a medium containing a BMP inhibitor and a TGF-β inhibitor;
(1b) subjecting the cells obtained in step (1a) to adherent culture performed on an extracellular matrix in a medium containing a BMP inhibitor, a TGF-β inhibitor, a SHH signal stimulant and FGF8;
(1c) subjecting the cells obtained in step (1b) to adherent culture performed on an extracellular matrix in a medium containing a BMP inhibitor, a TGF-β inhibitor, an SHH signal stimulant, FGF8 and a GSK-3β inhibitor; and
(1d) subjecting the cells obtained in step (1c) to adherent culture performed on an extracellular matrix in a medium containing BMP inhibitor and GSK-3β inhibitor.

The medium used herein may be prepared from a basal medium used for culturing animal cells. Examples of the basal medium include GMEM medium, IMDM medium, Medium 199 medium, Eagle's Minimum Essential Medium (EMEM), aMEM medium, Dulbecco's modified Eagle's Medium (DMEM) medium, StemFit medium, Ham's F12 medium, RPMI 1640 medium, Fischer's medium, Neurobasal Medium (Life Technologies; current name: Thermo Fisher), and mixture of these mediums. Preferably, GMEM medium is used. The medium may or may not contain serum. The medium may contain one or more serum substitutes such as albumin, transferrin, KnockOut Serum Replacement (KSR) (serum substitute), N2 Supplement, B-27 Supplement, a fatty acid, insulin, a collagen precursor, trace elements, 2-mercaptoethanol and 3'-thiol glycerol, as necessary; and may contain one or more substances such as a lipid, an amino acid, L-glutamine, Glutamax, a nonessential amino acid, a vitamin, a growth factor, a low molecular compound, an antibiotic substance, an antioxidant, pyruvate, a buffer and an inorganic salt. A preferable culture medium is GMEM medium containing KSR, 2-mercaptoethanol, a nonessential amino acid and pyruvate. A reagent selected from the group consisting of a BMP inhibitor, a TGF-β inhibitor, an SHH signal stimulant, FGF8 and a GSK-3β inhibitor may be added to this medium as appropriate to be used for culture.

Note that, the composition of a medium may be adjusted or changed during a process of culture as appropriate.

Adherent culture on an extracellular matrix may be performed by culturing using a culture vessel coated with the extracellular matrix. Coating treatment can be carried out by pouring a solution containing an extracellular matrix in a culture vessel, and then removing the solution as appropriate.

Step (1a) is usually carried out in a medium further containing a ROCK inhibitor. More specifically, step (1a) may be "subjecting pluripotent stem cells to adherent culture performed on an extracellular matrix in a medium containing a ROCK inhibitor, a BMP inhibitor and a TGF-β inhibitor".

In regard to the culture conditions, although not particularly limited, culture temperature is preferably about 37°C. Culture is carried out in a CO₂-containing atmosphere. The concentration of CO₂ is preferably about 2 to 5%.

The duration of culture is not particularly limited as long as it is a duration at which Corin-positive and/or Lrtml-positive cells emerge. Culture is preferably carried out in such a duration that the ratio of Corin-positive and/or Lrtml-positive cells contained in the cell population obtained after completion of step (1) becomes 10% or more. The culture is desirably carried out for at least 10 days and more preferably 12 days to 16 days.

As a plurality of pluripotent stem cells, pluripotent stem cells mutually dissociated may be used. Examples of a method for mutually dissociating cells include a mechanical dissociation method; and a dissociation method using a dissociation solution (for example, Accutase (trademark) and Accumax (trademark)) having a protease activity and a collagenase activity or a dissociation solution having a collagenase activity alone. Preferably, a method for dissociating human pluripotent stem cells by using trypsin or a trypsin alternative (for example, TrypLE CTS (Life Technologies; current name: Thermo Fisher)) is employed. If the cells are dissociated, it is desirable to add a ROCK inhibitor after dissociation as appropriate and then culture the resultant medium. If a ROCK inhibitor is added, the inhibitor is added and culture is carried out for at least a day, and more preferably for a day.

Note that, in an embodiment, human pluripotent stem cells (e.g., human iPS cells) may be subjected to adherent culture performed in a serum-free medium containing bFGF and an SHH signal stimulant in the absence of feeder cells, prior to step (1). The adherent culture is carried out in a cell vessel whose surface is coated with preferably laminin 511, E8 fragment of laminin 511 or vitronectin. The adherent culture is carried out by use of a feeder-free medium, preferably Essential 8, TeSR medium, mTeSR medium, mTeSR-E8 medium or StemFit medium, and further preferably, Essential 8 or StemFit medium (WO2017/183736).

### <Step (2)>

Step (2) includes suspending a plurality of cells obtained in step (1) in a continuous flow of a liquid vehicle, distinguishing neuronal precursor cells in the first differentiation stage, and separating the neuronal precursor cells in the first differentiation stage and other cells so as to let them flow into different continuous flows of the liquid vehicle.

In the present invention, in order to selectively separate neuronal precursor cells in the first differentiation stage from the plurality of cells obtained in step (1), the neuronal precursor cells are distinguished based on a predetermined index. The index used herein is not particularly limited, and an index well known to those skilled in the art may be used as appropriate. More specifically, marker gene/protein expressed specifically in the neuronal precursor cells in the first differentiation stage, size of the cells, density of the cells, or the like may be used.

When the marker expressed specifically in the neuronal precursor cells is used as the index, marker-positive cells may be separated by use of a substance that binds specifically to the marker, and by use of a cell sorter.

As the marker, a protein expressed on the surface of desired neuronal precursor cells in the first differentiation stage may be used. As the substance that specifically binds to the marker, an antibody or an aptamer may be used, and preferably, an antibody or an antigen-binding fragment thereof may be used.

The antibody may be a polyclonal or monoclonal antibody. These antibodies may be prepared by a technique well known to those skilled in the art (Current protocols in Molecular Biology edit. Ausubel et al. (1987) Publish. John Wiley and Sons. Section 11. 12-11. 13). More specifically, when the antibody is a polyclonal antibody, the protein of the marker expressed in *Escherichia coli* or mammalian cell line in accordance with a routine method, an oligopeptide having a partial amino acid sequence of the marker, or a glycolipid is purified, and then, a non-human animal such as a rabbit is immunized with the above purified substance. In this manner, the polyclonal antibody can be obtained from the serum of the immunized animal in accordance with a routine method. On the other hand, in the case of a monoclonal antibody, the monoclonal antibody can be obtained from a hybridoma cells prepared by fusing spleen cells taken from the non-human animal immunized as mentioned above with myeloma cells (Current protocols in Molecular Biology edit. Ausubel et al. (1987) Publish. John Wiley and Sons. Section 11.4-11.11). An example of an antigen-binding fragment of an antibody is a part of the antibody (for example, Fab fragment) or a synthetic antibody fragment (for example, single-chain Fv fragment "ScFv"). An antibody fragment such as Fab and F(ab)₂ fragments may be prepared in accordance with a method well known in the field of genetic engineering.

In order to recognize or separate the cells expressing a marker, the substance that binds to the marker may be bound or joined, for example, to a detectable substance such as a fluorescent label, a radioactive label, a chemiluminescent label, an enzyme, biotin or streptavidin, or to a substance that enables isolation and extraction, such as protein A, protein G, beads or magnetic beads.

The substance that binds to the marker may be indirectly labeled. Indirect labeling may be performed in accordance with various methods known to those skilled in the art, and for example, a method using an antibody (secondary antibody) that specifically binds to the antibody and is labeled in advance may be used.

In the present specification, an aptamer that binds specifically to a marker may be produced by a technique well known to those skilled in the art (SELEX method (systematic evolution of ligand by exponential enrichment): Ellington, A. D. & Szostak, J.W. (1990) Nature, 346, 818-822., Tuerk, C. & Gold, L. (1990) Science, 249, 505-510).

When the neuronal precursor cells in the first differentiation stage are the neuronal precursor cells committed to the midbrain floor plate, Corin and/or Lrtm1 may be used as a marker. The sequence of human Corin may be obtained based on NCBI accession number NM 006587. Similarly, the sequence of human Lrtm1 may be obtained based on NCBI accession number NM 020678. For example, the antibody to Corin may be obtained by a production method described in WO2004/065599 and WO2006/00924, and the antibody to Lrtm1 may be obtained by a production method described in WO2013/015457.

The cell separator to be used in step (2) has a mechanism by which a plurality of cells obtained in step (1) are suspended in a continuous flow of a liquid vehicle; the neuronal precursor cells in the first differentiation stage are distinguished; and the neuronal precursor cells in the first differentiation stage are separated from other cells so as to let them flow into different continuous flows of the liquid vehicle.

In the present specification, a cell separator (also referred to as a cell sorter) is an apparatus equipped with a device for detecting an index characteristic to neuronal precursor cells in the first differentiation stage, such as a marker, and with a liquid channel through which liquid can be continuously fed without forming liquid droplets. Cells can be separated in a continuous solution system without forming liquid droplets by use of this cell separator.

In the present specification, a cell separator is preferably a completely closed system. More specifically, the cell separator may be a microfluidic-channel system cell sorter described in a literature written by Hulspas R, et al., Cytotherapy. 2014 Oct; 16 (10): 1384-9 (Hulspas literature). The cell separator of this literature is a completely closed microfluidic-channel system, and it enables separation of cells without forming liquid droplets. As the cell separator, a separator that can separate cells at a high speed (for example, process about 5000 particles or more/second, and ten-million cells or more, in total, per operation) is preferable.

More specifically, Gigasort cell sorter manufactured by Cytonome may be used (see, https://www.ncbi.nlm.nih.gov/pubmed/25065635 (Hulspas literature) and http://www.cytonome.com/). This cell sorter is a completely closed microfluidic-channel system, and the cells can be separated in continuous solution system without forming liquid droplets by bending a flow channel of cells to be separated with air pressure, after the cells are passed through a detector of a marker or the like.

### <Step (3)>

Step (3) is a step of culturing the neuronal precursor cells in the first differentiation stage separated in step (2) in the presence of a second differentiation-inducing factor to obtain a mixture of adherent cell populations. The mixture of adherent cell populations contains 50% or more of adherent cell population having the following characteristics (b1) and (b2), based on a total number of adherent cell populations:
(b1) containing neural cells in a second differentiation stage; and
(b2) containing 1000 or more cells.

In the present specification, neural cells in the second differentiation stage refer to cells, which are selected and separated in step (2) and continued to be cultured to be in a further advanced differentiated stage, and include precursor cells committed to differentiate into predetermined neural cells. The neural cells in the second differentiation stage are not particularly limited as long as the cells are in a more advanced differentiation stage than the neuronal precursor cells in the first differentiation stage. The degree of differentiation varies depending on the desired neural cells.

The neural cells in the second differentiation stage may be neuronal cells positive for at least one, preferably at least two, further preferably at least three of TUJ1, OTX2, FOXA2, LMX1A, LMX1B, En1, Nurr1, PITX3, DAT, GIRK2 and TH. An embodiment of the neural cells in the second differentiation stage may be FOXA2-positive and/or TUJ1-positive cells.

Preferably, the neural cells in the second differentiation stage are ventral midbrain-derived neuronal cells, and more specifically, may be dopaminergic neuron progenitor cells or dopaminergic neural cells. The neural cells in the second differentiation stage are preferably FOXA2-positive and TUJ1-positive dopaminergic neuron progenitor cells.

As a method for inducing differentiation of the cells obtained in step (2) into neural cells in the second differentiation stage, a method known to those skilled in the art may be used as appropriate, depending on the type of neural cells desired. More specifically, culture may be carried out in an appropriate culture medium in the presence of a second differentiation-inducing factor well known to those skilled in the art. The second differentiation-inducing factor herein refers to a factor having an influence on differentiation state (expression of transcription factors, genes, or proteins involved in differentiation) of cells, and examples thereof include a low molecular compound, a protein, a peptide fragment of a protein, and a physical factor such as carbon dioxide gas, oxygen partial pressure or pressure. For example, in the case of dopaminergic neuron progenitor cells, a known method described in Stem cells reports, vol. 2 337-350, 2014 may be used.

A method for inducing differentiation will be more specifically described by way of the case where the neural cells in the second differentiation stage are neuronal cells including dopaminergic neuron progenitor cells.

The medium used herein may be prepared from a basal medium used for culturing animal cells. Examples of the basal medium include GMEM medium, IMDM medium, Medium 199 medium, Eagle's Minimum Essential Medium (EMEM), aMEM medium, Dulbecco's modified Eagle's Medium (DMEM) medium, Ham's F12 medium, RPMI 1640 medium, Fischer's medium, Neurobasal Medium (Life Technologies Corporation; current name: Thermo Fisher), and a mixture of these mediums. Preferably, Neurobasal Medium is used. The culture medium may or may not contain serum. The medium may contain one or more serum substitutes such as albumin, transferrin, KnockOut Serum Replacement (KSR) (serum substitute for FBS during culture of ES cells), N2 Supplement, B-27 Supplement, a fatty acid, insulin, a collagen precursor, trace elements, 2-mercaptoethanol and 3'-thiol glycerol, as necessary; and may contain one or more substances such as a lipid, an amino acid, L-glutamine, Glutamax, a nonessential amino acid, a vitamin, a growth factor, a low molecular compound, an antibiotic substance, an antioxidant, pyruvate, buffer, an inorganic salt, and a nucleic acid (for example, dibutyryl cyclic AMP (dbcAMP)). A preferable culture medium is Neurobasal Medium containing B-27 Supplement, ascorbic acid and dbcAMP. A neurotrophic factor may be added to this medium as appropriate to be used for culture.

Induction of differentiation may be carried out in suspension culture. The suspension culture herein means that cells are cultured without being adhered to a culture vessel. Although it is not particularly limited, suspension culture may be carried out by using a culture vessel to which no artificial treatment (for example, coating with an extracellular matrix) for improving adhesiveness to cells is applied, or a culture vessel to which a treatment (for example, coating treatment with polyhydroxyethyl methacrylate (poly-HEMA), a nonionic surfactant polyol (Pluronic F-127 or the like), or a phospholipid-like structure (for example, a water soluble polymer (Lipidure) having 2-methacryloyloxyethyl phosphorylcholine as a structural unit)) for suppressing adhesion is artificially applied.

In regard to culture conditions, although not particularly limited, culture temperature is about 30 to 40°C and preferably about 37°C. Culture is carried out in a CO₂-containing atmosphere. The concentration of CO₂ is preferably about 2 to 5%.

The duration of culture is not particularly limited as long as it is a duration at which FOXA2-positive cells emerge. Culture is desirably carried out at least for 7 days, more preferably 7 days to 30 days, further preferably 14 days to 21 days, 14 days to 20 days, 14 days to 18 days, or 14 days to 16 days, and most preferably 16 days.

Culture is desirably carried out with a ROCK inhibitor added as appropriate. If a ROCK inhibitor is added, the inhibitor is added and culture is carried out for at least a day, and more preferably for a day.

### IV. Adherent cell population and mixture thereof

Owing to a method for producing a mixture of adherent cell populations, it is possible to produce a mixture of adherent cell populations containing 50% or more of adherent cell populations having the following characteristics (b1) and (b2), based on the total number of adherent cell populations:
(b1) containing neural cells in a second differentiation stage; and
(b2) containing 1000 or more cells.

Furthermore, the adherent cell populations having characteristics (b1) and (b2) may be obtained from the mixture of adherent cell populations obtained by the above method for producing a mixture of adherent cell populations, by a method for producing an adherent cell population including separating the adherent cell population having characteristics (b1) and (b2).

The mixture of adherent cell populations may be a mixture of three-dimensional adherent cell populations (more specifically, a mixture of cell aggregates) or a mixture of adherent cell populations in the form of a two dimensional single or multiple layer (more specifically, a cell sheet). The three-dimensional adherent cell population may have an equivalent circle diameter of 100 µm to 2000 µm, preferably 100 µm to 1000 µm, further preferably, 200 µm to 600 µm and further preferably, 300 µm to 600 µm.

During the culture of the adherent cell population or a mixture thereof, cell death can be suppressed. When the adherent cell population is cultured for 14 to 20 days, the number of cells at the completion of culture is 5% or more, preferably 8% or more, further preferably 10% or more, further preferably 15% or more, further preferably 60% or more, and further preferably about 100% of the cells at the beginning of the culture.

Note that, the change in the number of cells by cultured varies depending on the type of cell. In a case where the neural cells in the second differentiation stage are dopaminergic neuron progenitor cells, it is known that usually about 80% or more of the cells die. However, when the neural cells in the second differentiation stage are cultured by the production method of the present invention for 14 to 20 days, the number of cells at the completion of the culture is 5% or more, preferably 8% or more, further preferably 10% or more, further preferably 15% or more, and further preferably 20% or more, and more specifically, for example 15% to 80% or 15% to 50% of the number of cells at the beginning of the culture.

On the other hand, if neural cells in the second differentiation stage are neural stem cells, it is known that, usually, the number of cells once decreases but then increases back. In the case of such neural cells, when the cells in the second differentiation stage are cultured for 14 to 20 days, the number of cells at the completion of the culture is 80% or more or about 100% of the number of cells at the beginning of the culture.

One embodiment of the three-dimensional adherent cell population is a cell aggregate. Preferably, the cell aggregate further has following characteristics:
(b3) a convexity or a solidity is 0.5 or more, preferably 0.7 to 1.0, and further preferably 0.8 to 1.0;
(b4) Feret diameter ratio is 0.5 or more, preferably 0.6 to 1.0, and further preferably 0.7 to 1.0; and
(b5) a circularity is 0.3 or more, preferably 0.5 to 1.0, and further preferably 0.7 to 1.0.

A preferable embodiment includes a cell aggregate having the following characteristics:
- an equivalent circle diameter is 100 µm to 1000 µm;
- a convexity or a solidity is 0.8 to 1.0;
- a Feret diameter ratio is 0.7 to 1.0; and
- a circularity is 0.7 to 1.0.

The cell aggregate further preferably has the following characteristics:
In the mixture of cell aggregates to be obtained, at least one index selected from the group consisting of a circularity, a minimum diameter, a maximum diameter, a vertical Feret diameter or a horizontal Feret diameter, a Feret diameter ratio, an equivalent circle diameter, a perimeter, an area, and a convexity or a solidity has a coefficient of variation of 15% or less.

In the above production method, starting stem cells are not particularly limited as long as they can be differentiated into neural cells, and are preferably, pluripotent stem cells, neural stem cells, mesenchymal stem cells or Muse cells.

The stem cells are further preferably pluripotent stem cells, and further more preferably ES cells or iPS cells.

The adherent cell population obtained by the production method of the present invention is also a concept of the present invention.

The neuronal precursor cells obtained in step (2) of the production method constitute a non-adherent cell population, namely a mixture of mutually discrete cells, that can be induced to differentiate into the cell aggregate or the adherent cell population of the present invention by culturing them in the presence of a second differentiation-inducing factor. This mixture of cells also falls within the scope of the present invention.

More specifically, an example includes a mixture of cells that can be induced to differentiate into the cell aggregate and adherent cell population of the present invention that may be obtained by culturing the cells including about 70% or more of Corin-positive or Lrtml-positive cells in the presence of a second differentiation-inducing factor.

A cell aggregate of the neural cells in the second differentiation stage of the present invention can be obtained by subjecting the mixture of the cells to suspension culture. Also, by subjecting the mixture of the cells to adherent culture, a single-layer cell sheet can be produced. This cell sheet also falls within the scope of the present invention.

### V. Pharmaceutical composition

The cell aggregate or the mixture thereof or the adherent cell population of the present invention is useful as a pharmaceutical composition for transplantation for a patient with a disease in need of transplantation of neuronal cells or neural cells that can be differentiated into neuronal cells, and can be used as a medicament such as a therapeutic drug for a disease associated with degeneration, damage or dysfunction of neuronal cells. Namely, a pharmaceutical composition containing the cell aggregate or adherent cell population of the present invention and a pharmaceutically acceptable carrier also fall within the scope of the present invention.

Examples of the disease in need of transplantation of neuronal cells or the disease associated with damage or dysfunction of neuronal cells include spinal cord injury, motor neuropathy, multiple sclerosis, amyotrophic lateral sclerosis, atrophic lateral sclerosis, Huntington's chorea disease, multiple system atrophy, spinocerebellar degeneration, Alzheimer's disease, retinitis pigmentosa, age-related macular degeneration and Parkinson's syndrome, and Parkinson's disease is preferable.

One embodiment of the present invention is a therapeutic drug for Parkinson's disease containing the cell aggregate or the mixture thereof or the adherent cell population of the present invention containing dopaminergic neuron progenitor cells. The number of dopaminergic neuron progenitor cells contained in the therapeutic drug for Parkinson's disease is not particularly limited as long as the graft administered can be engrafted, and for example, 1.0 × 10⁴ cells or more may be contained per transplantation. The number of cells may be increased or decreased as appropriate, depending on the symptom and the body size. Dopaminergic neuron progenitor cells may be transplanted to a disease site by a technique described, for example, in Nature Neuroscience, 2, 1137 (1999) or N Engl J Med. 344: 710-9 (2001).

A pharmaceutically acceptable carrier is not particularly limited as long as it is a substance used for maintaining survival of cells, and substance well known to those skilled in the art may be used. More specifically, a physiological aqueous solvent (saline, buffer, serum free medium, or the like) may be used. A preservative, a stabilizer, a reductant, a tonicity agent, or the like that is routinely used in medicament containing tissues or cells to be transplanted used in transplantation therapy may be added as necessary.

The pharmaceutical composition of the present invention may be prepared as a cell suspension by suspending the cell aggregate or the mixture thereof, or the adherent cell population according to the present invention in an appropriate physiological aqueous solvent. If necessary, the cell suspension may be cryopreserved by adding a cryopreservation agent to the suspension, which may be thawed just before use, washed, and used for transplantation.

### VI. Treatment method

One embodiment of the present invention is a method for treating a disease in need of supplement of neural cells, comprising transplanting the cell aggregate or the mixture thereof, or the adherent cell population of the present invention to a patient with a disease in need of transplantation of neural cells.

As an embodiment of the present invention, the cell aggregate or the mixture thereof, or the adherent cell population containing dopaminergic neuron progenitor cells, obtained in the present invention may be administered to a patient with Parkinson's disease as a preparation, more specifically, as a preparation for transplantation. This can be performed by suspending the dopaminergic neuron progenitor cells obtained in saline or the like, and transplanting the cells to a region, for example, striatum, of a patient lacking in dopamine nerve.

### VII. Transplantation

Upon transplantation, the cell aggregate of the present invention may be preserved in a medium necessary for maintaining viability of the cell aggregate. The "medium necessary for maintaining viability of the cell aggregate" may be a culture medium, a physiological buffer, or the like, but are not particularly limited as long as a cell population containing dopaminergic neuron progenitor cells is kept alive, and may be selected by those skilled in the art as appropriate. As an example, a culture medium prepared from a basal medium routinely used for culturing animal cells may be used. Examples of the basal medium include mediums that can be used for culturing animal cells, such as BME medium, BGJb medium, CMRL 1066 medium, GMEM medium, Improved MEM Zinc Option medium, Neurobasal medium, IMDM medium, Medium 199 medium, Eagle MEM medium, aMEM medium, DMEM medium, F-12 medium, DMEM/F12 medium, IMDM/F12 medium, Ham's medium, RPMI 1640 medium and Fischer's medium, or a mixture of these mediums.

In the present specification, "engraftment" means that the cells transplanted survive *in vivo* for a long term (e.g., 30 days or more, 60 days or more, 90 days or more), adhere to the organs, and remain there.

In the present specification, "functional engraftment" refers to a state where the cells transplanted are engrafted and play their original role *in vivo.*

In the present specification, "functional engraftment rate" refers to the ratio of cells functionally engrafted in the transplanted cells. The functional engraftment rate of the dopaminergic neuron progenitor cells transplanted may be obtained, for example, by counting the number of TH-positive cells in a graft.

The functional engraftment rate of the transplanted cells (including dopaminergic neuron progenitor cells and dopaminergic neuron progenitor cells induced after transplantation) obtained by transplanting the above cell aggregate is 0.1% or more, preferably 0.2% or more, further preferably 0.4% or more, further preferably 0.5% or more, and further preferably 0.6% or more.

In the present specification, examples of a mammal serving as a target for transplantation include a human, a mouse, a rat, a guinea pig, a hamster, a rabbit, a cat, a dog, a sheep, a pig, a cow, a horse, a goat and a monkey, and a mammal is preferably a rodent (e.g., a mouse or a rat) or a primate (e.g., human or monkey), and more preferably a human.

### Examples

The present invention will be more specifically described by way of the following Examples; however, the present invention is not limited by these.

### (Experiment 1)

### <Cells and culture>

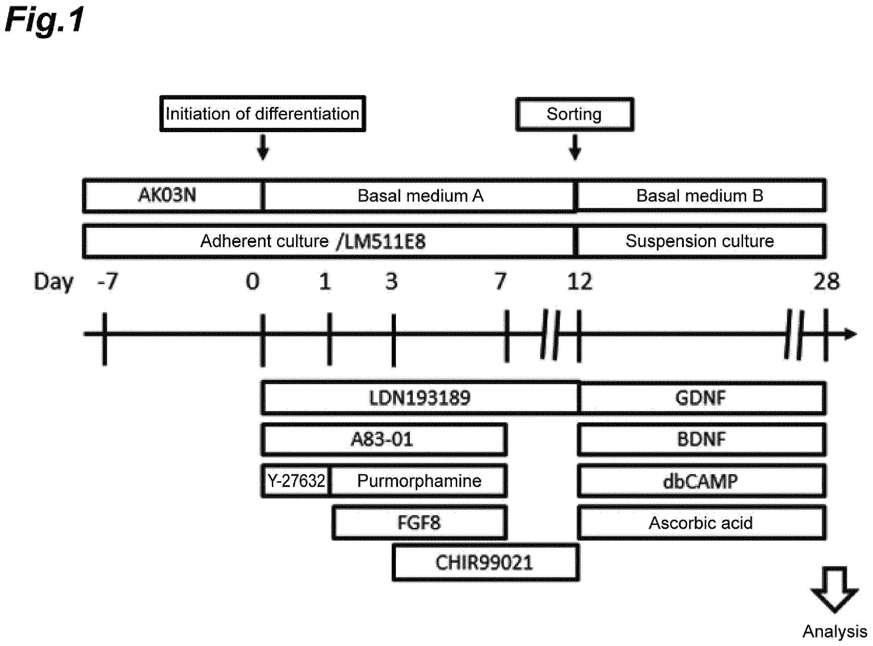

A protocol for inducing differentiation of human iPS cells into dopaminergic neuron progenitor cells is shown in Figure 1. Culture conditions of expansion culture up to initiation of differentiation induction (day -7 to 0), a first differentiation stage from the initiation of differentiation induction to the 12th day (day 0 to 12), and the second differentiation stage from the 12th day after initiation of differentiation induction to the 28th days (day 12 to 28) are shown in Figure 1. Note that, sorting was carried out on the 12th (day 12) day after initiation of differentiation induction.

Human iPS cells, QHJ-I01, which were obtained by introducing Oct3/4, Sox2, Klf4, L-MYC, LIN28 and p53 dominant negative body (Okita, K., et al. Stem Cells 31, 458-66, 2013) into human PBMC by use of an episomal vector, were received from prof. Yamanaka, et al., of Kyoto University.

The iPS cells were cultured by a method according to the description of Miyazaki T, et al., Nat Commun. 3: 1236, 2012. Briefly, iPS cells were subjected to maintenance culture performed on a 6-well plate coated with Laminin-511E8, in an undifferentiation-maintaining medium (AK03N) containing FGF2 (bFGF).

The cell population obtained by maintenance culture of iPS cells was dissociated by use of TrypLE CTS (Life Technologies), and seeded at 5 × 10⁶ cells per well to a separately prepared 6-well plate coated with Laminin-511E8 (iMatrix-511, Nippi), and then, the medium was exchanged with a differentiation medium (initiation of differentiation induction: day 0). The differentiation medium was prepared by adding 10 µM Y-27632 (WAKO), 0.1 µM LDN193189 (STEMGENT) and 0.5 µM A83-01 (WAKO) to basal medium A. Note that, basal medium A is GMEM (Invitrogen) containing 8% KSR (Invitrogen), 1 mM sodium pyruvate (Invitrogen), 0.1 mM MEM nonessential amino acid (Invitrogen) and 0.1 mM 2-mercaptoethanol (WAKO). Next day (day 1), the medium was exchanged with basal medium A containing 0.1 µM LDN193189, 0.5 µM A83-01, 2 µM Purmorphamine (WAKO) and 100 ng/mL FGF8 (WAKO). Two days later (day 3), the medium was exchanged with basal medium A containing 0.1 µM LDN193189, 0.5 µM A83-01, 2 µM Purmorphamine, 100 ng/mL FGF8 and 3 µM CHIR99021 (WAKO). Four days later (day 7), the medium was exchanged with basal medium A containing 0.1 µM LDN193189 and 3 µM CHIR99021. During these periods, the medium was exchanged once per day. On the 12th day (day 12) after initiation of differentiation induction, cell sorting using an anti-Corin antibody was carried out.

### <Sorting pretreatment>

Five days after the culture in basal medium A containing 0.1 µM LDN193189 and 3 µM CHIR99021, in other words, the 12th day (day 12) after initiation of differentiation induction, the cells were dissociated by use of TrypLE CTS, and suspended in Ca2⁺Mg2⁺-free HBSS (Invitrogen) containing 2% FBS, 30 µM Y-27632 (WAKO), 20 mM D glucose and 50 µg/mL penicillin/streptomycin. The above anti-Corin antibody was added, and incubation was carried out at 4°C for 20 minutes. Fluorescence-activated cell sorting (FACS) was carried out to recover Corin-positive cells, which were subjected to various analyses.

Note that, an anti-Corin antibody was prepared by the following method. Of cynomolgus monkey Corin genes, a gene sequence encoding a part (79-453 amino acids) of an extracellular region was introduced into 293E cells to allow the extracellular region fragment of Corin protein to be expressed and collected. Mice were immunized with the protein collected, and then, lymphocytic cells were taken out and fused with myeloma cells. From the fused cell population, a clone responding to Corin was selected. The culture supernatant of the clone was used as an anti-Corin monoclonal antibody after a fluorescent label was attached.

### <Sorting>

As a cell sorter for FACS, a Stream-In-Air system sorter FACSJazz (trademark) (company: BD) or a micro-channel system sorter Gigasort (company: Cytonome) was used. Corin-positive cells were collected and subjected to various analyses.

As sorting conditions in the case of FACSJazz (trademark), a nozzle diameter of 100 µm and a sheath pressure of 29 PSI, which are routinely used for sorting neuronal cells, were employed. As sorting conditions in the case of Gigasort, the channel inner diameter of about 200 µm and a sheath pressure of 14-20PSI, which are the manufacturer's standard, were employed.

### <Suspension culture after sorting>

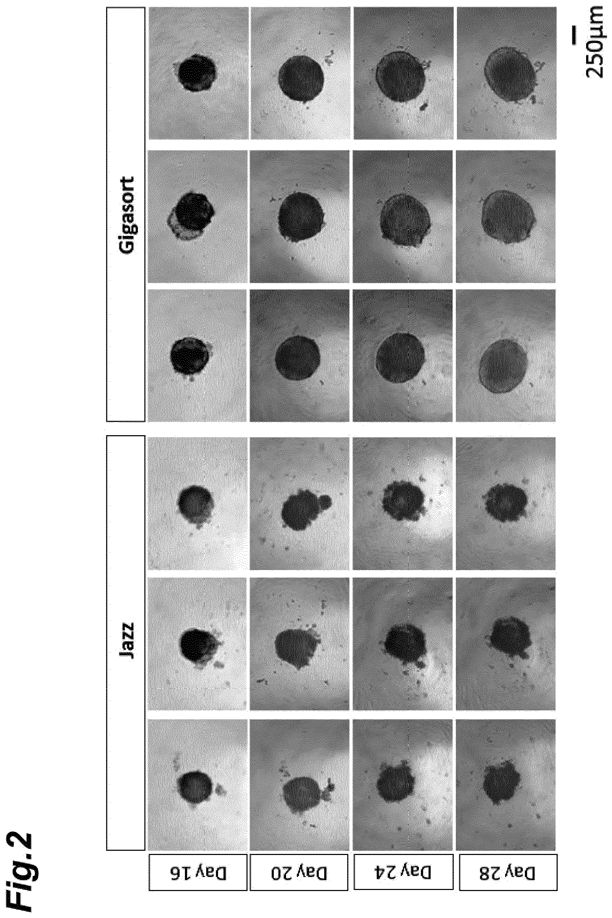

The Corin-positive cells collected were transferred at 20000 cells/well to a PrimeSurface 96U plate (Sumitomo Bakelite Co., Ltd.), and subjected to suspension culture using basal medium B (Neurobasal (registered trademark) medium (Invitrogen) containing B-27 (trademark) Supplement minus vitamin A (Invitrogen), 20 ng/mL BDNF (WAKO), 10 ng/mL GDNF (WAKO), 200 mM Ascorbic acid (WAKO) and 0.4 mM dbcAMP (Sigma)). A medium containing 30 µM Y-27632 was used as a first culture medium, and a culture medium without Y-27632 was used when a half of the culture medium was exchanged once in three days. Suspension culture was carried out up to the 16th day after sorting (day 28 after completion of differentiation induction) to obtain dopaminergic neuron progenitor cells by differentiation induction. During this period, cell aggregates in the suspension culture were photographed by a microscope every 4 days. The images observed are shown in Figure 2.

In the case where cell aggregates were sorted by Jazz, the size of cell aggregates in suspension culture did not change from the 16th day to the 28th day (day 16 to day 28) after initiation of differentiation induction. In contrast, in the case where cell aggregates were sorted by Gigasort, it was found that the diameter of cell aggregates started to increase from around the 20th day (day 20) after initiation of differentiation induction. Furthermore, on all of day 16, day 20, day 24 and day 28, more dead cells, debris and satellite-like cell population were observed for cell aggregates sorted by Jazz compared to the cell aggregates sorted by Gigasort. For example, the 3rd aggregate from the left on "day 16" of the case in which Jazz was used, not only cell aggregates but also small black grains (namely, satellite-like cell population) and debris surrounding the cell aggregate were observed. In contrast, for the case in which Gigasort was used, debris and satellite-like cell population were significantly less. When the cell aggregates of the group sorted by Gigasort were observed, the borderlines of cell aggregates were clear, and the formation of a debris layer, which was observed around the cell aggregates sorted by Jazz, and small cell populations present in a satellite manner were not observed. It was found that the numbers of dead cells and cell populations of dead cells present around the cell aggregates were low. Furthermore, the cell aggregates derived from Gigasort on and after day 24 had a diameter of about 450 µm to about 600 µm, which was large, compared to cell aggregates (outer edge was unclear, and the diameter of the cell aggregates excluding debris part was about 350 µm to about 400 µm) derived from Jazz.

### <Cell count>

On Day 28, the cell aggregates (the number is shown in Table 1), together with a culture medium, were collected from a 96-well U bottom plate with a micro-pipettor, and cell aggregates were allowed to precipitate by gravity. The supernatant of the medium was removed, and 1 mL of PBS was added. The cell aggregates were allowed to precipitate by gravity. The supernatant was removed, and 1 mL of the enzyme solution of the neuronal cell dispersion kit was added. Incubation was carried out at 37°C in a water bath. The cell suspension was pipetted up and down every 10 minutes, and at the timepoint of 30 minutes after initiation of incubation, 10 µL of the cell suspension was collected, mixed with 10 µL of trypan blue (Thermo Fisher Scientific) and injected into a hemocytometer. The number of cells was counted under the microscope. The results are shown in Table 1, the column "in enzyme solution". Also, the ratio of trypan blue non-positive cells/total number of cells was calculated, which was regarded as a cell survival rate. Subsequently, the dispersion liquid and removal liquid of the neuronal cell dispersion kit were added and centrifugation was carried out. After the supernatant was removed, resuspension with 1 mL of PBS was carried out. Then, 10 µL of the resuspension solution was mixed with trypan blue (Thermo Fisher Scientific) and injected into a hemocytometer. The number of cells was counted under the microscope. The results are shown in Table 1, the column of "after washing [hemocytometer]". Moreover, a resuspended sample was subjected to the measurement by an automatic cell counter (Chemometec, NC-200). The results are shown in Table 1, the column "after washing [NC-200]".

**[Table 1]**

| Measurement results | | Jazz | Gigasort |
|---|---|---|---|
| Number of cell aggregates | | 480 Cells | 438 Cells |
| In enzyme solution [hemocytometer] | Vial cells (cells/mL) | 1.4×10⁶ | 3.8×10⁶ |
| | Dead cells (cells/mL) | 0.0 | 0.0 |
| | Survival rate (%) | 100 | 100 |
| | Number of cells/ cell aggregates | 2,813 | 8,562 |
| After washing [hemocytometer] | Vial cells (cells/mL) | 1.3×10⁶ | 3.0×10⁶ |
| | Dead cells (cells/mL) | 0.0 | 0.0 |
| | Survival rate (%) | 100 | 100 |
| | Number of cells/ cell aggregates | 2,604 | 6,735 |
| After washing [NC-200] | Vial cells (cells/mL) | 1.4×10⁶ | 4.0×10⁶ |
| | Dead cells (cells/mL) | 4.8×10³ | 7.7×10³ |
| | Survival rate (%) | 100 | 100 |
| | Number of cells (Cells)/ cell aggregates | 2,813 | 9,064 |

As shown in Table 1, it was found that, with any measurement methods, the number of cells per cell aggregate of cell aggregates of the group sorted by Gigasort was about three times as large as that of the cell aggregates of the group sorted by Jazz. Note that, the survival rate at the time of measuring of the number of cells were all 100 percent.

### <Cell morphometry>

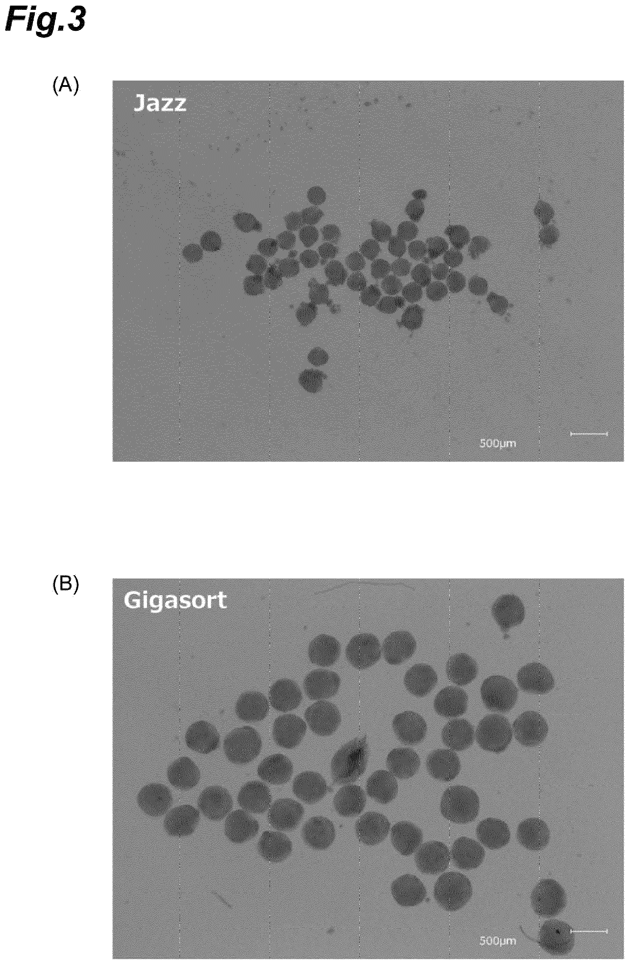

On Day 28, 48 cell aggregates, together with a culture medium, were collected from a 96-well U bottom plate with a micro-pipettor, and transferred to a 6-cm low-adhesive dish (Sumitomo Bakelite Co., Ltd.). The cell aggregates were photographed by transillumination by use of a digital microscope (KEYENCE CORPORATION; VHX-5000) to obtain the images shown in Figure 3. The number of cell aggregates of the group sorted by Gigasort within the field of view was 47 (B), and those by Jazz was 48 (A).

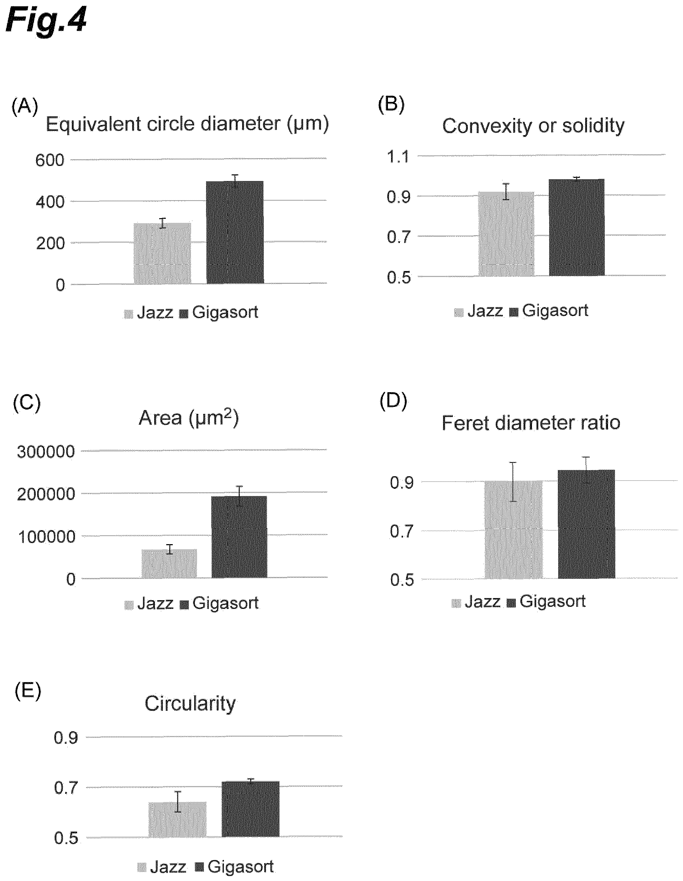

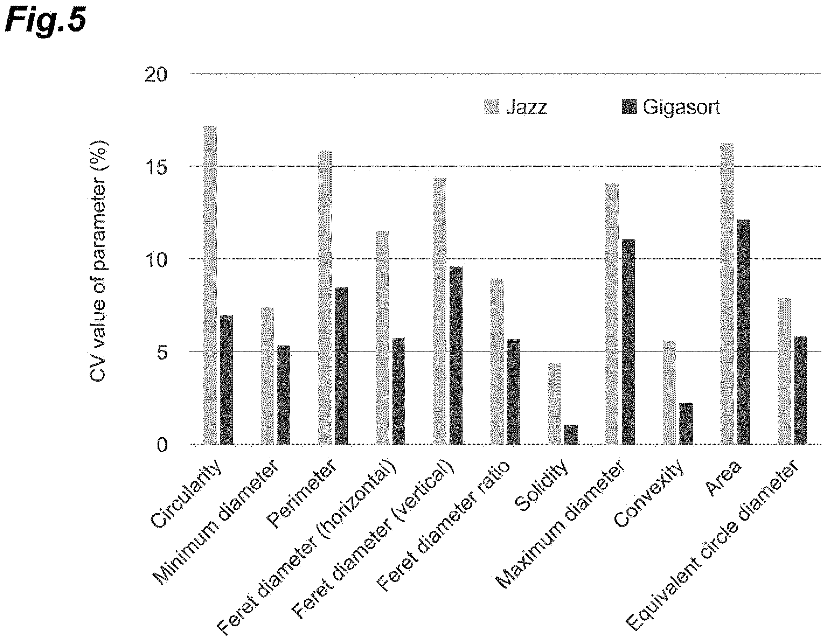

The images thus obtained were analyzed with VHX-5000 (Ver 1.3.2.4) software installed in the digital microscope, and the circularity, minimum diameter, perimeter, Feret diameter (horizontal), Feret diameter (vertical), Feret diameter ratio, solidity, maximum diameter, convexity, area and equivalent circle diameter of cell aggregates were measured (Figure 4). Among them, comparison of the equivalent circle diameter, convexity or solidity, area, Feret diameter ratio and circularity between Jazz (light gray) and Gigasort (dark gray) are shown in the graphs of Figure 4. From the data obtained, standard deviations and coefficients of variation (CV values) were calculated. The CV values are shown in Figure 5.

As shown in Figure 3, it was found that the cell aggregates sorted by Gigasort were large also in visua,l compared to the cell aggregates sorted by Jazz. As shown in Figure 4, compared to the cell aggregates sorted by Jazz, the cell aggregates sorted by Gigasort had larger equivalent circle diameter and area, and variation of convexity or solidity, which indicates the presence of chips and protrusions and which serves as an index for smoothness of circumference of a sphere, was remarkably small.

From these results, it was shown that by sorting cells using Gigasort, more cells can be kept alive with little damage, and cell aggregates formed of these cells were larger and close to a true sphere, and were a smooth sphere.

The coefficients of variations (CV value) of each parameter was calculated. As a result, as shown in Figure 5, it was found that CV values of all parameters such as size (minimum diameter, perimeter, Feret diameter, Feret diameter ratio, maximum diameter, area and equivalent circle diameter), sphere shape (circularity), and surface condition (convexity or solidity) were small in the cell aggregates of the group sorted by Gigasort, compared to the cell aggregates of the group sorted by Jazz. Namely, it was found that the cell aggregates of a group sorted by Gigasort were highly uniform.

### <Flow cytometry analysis>

On Day 28, an enzyme solution was added to the cells and the cells were dispersed to prepare a sample for counting cell number. To the sample, a dispersion liquid and a removal liquid were added, and the resultant mixture was centrifuged. The supernatant was removed, and the pellet was resuspended in PBS and stained with Live/Dead reagent (Thermo Fisher Scientific), Foxa2 (R&D)/Alexa647-anti-goat (Thermo Fisher Scientific), Alexa488-Tuj 1 (BD), Alexa647-Oct3/4 (BD), FITC-TRA2-49 (Millipore), PerCP-Cy5.5-Sox1 (BD), Alexa647-Pax6 (BD) and Alexa488-Ki67 (BD). The ratio of FOXA2-positive and TUJ1-positive cells, FOXA2-positive cells, or TUJ1-positive cells to the whole cells contained in the cell suspension was calculated using a flow cytometer Gallios (Beckman coulter) (Table 2). In either one of the cases of using Jazz and Gigasort, the positive rates for FOXA2 and/or TUJ1 marker were high, whereas the positive rates for OCT3/4 and/or TRA-2-49 serving as pluripotency markers, were low.

**[Table 2]**

| Evaluation item | Jazz | Gigasort |
|---|---|---|
| | Positive rate (%) | Positive rate (%) |
| FOXA2/TUJ1 | 86.1 | 85.1 |
| FOXA2 | 97.4 | 95.3 |
| TUJ1 | 87.2 | 88.8 |
| OCT3/4/TRA-2-49 | 0.0 | 0.0 |
| OCT3/4 | 0.5 | 0.4 |
| TRA-2-49 | 0.0 | 0.0 |

From Table 2, it was found that, in the cells sorted by Gigasort and subjected to maturation culture, the positive rates for expressed genes were the same as those in the cell group sorted by Jazz.

### <Immunostaining>

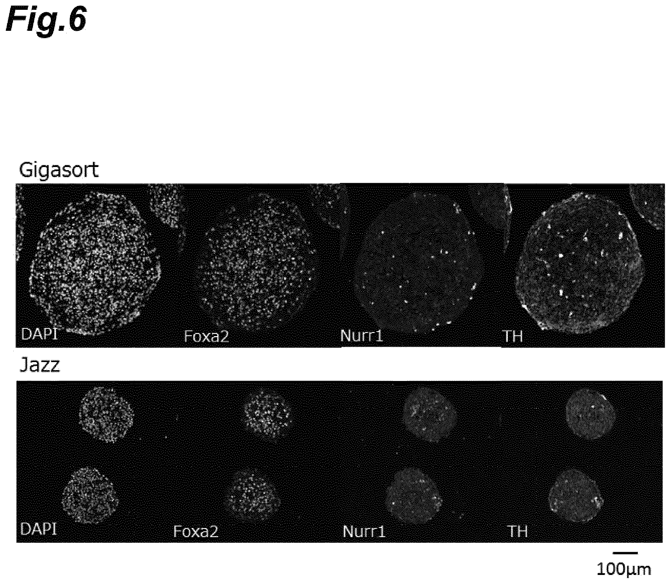

On Day 28, 10 cell aggregates, together with a culture medium, were collected from a 96-well U-bottom plate with a micro-pipettor, and cell aggregates were allowed to precipitate by gravity. The supernatant of the medium was removed, and 1 mL of PBS was added. The cell aggregates were allowed to by gravity. The supernatant was removed and the cell aggregates were fixed with PFA, embedded with an OCT compound and frozen. Then, the cell aggregates were sliced to 10 µm by using a cryostat (Leica). The sections were attached onto glass slides, blocked with a blocking buffer (2% normal donkey serum, 0.3% TritonX100/PBS), primarily stained with an anti-Nurrl mouse IgG antibody (Perseus Proteomics), an anti-Foxa2 goat IgG antibody (R&D systems) and an anti-THrabbit IgG antibody (Millipore), and then, secondarily stained with Alexa488 labeled anti-mouse antibody, Alexa594 labeled anti-goat antibody, Alexa647 labeled anti-rabbit antibody and DAPI (all were provided by Thermo Fisher Scientific). The sections stained were enclosed by use of VECTASHIELD Hard set, and were observed by a confocal microscope (Olympus FV1200) (Figure 6).

It was found that expression levels of markers of the cells sorted by Gigasort and subjected to maturation culture did not significantly differ to the cell group sorted by Jazz. In other words, the degrees of differentiation were almost the same.

### Industrial Applicability

The present invention is useful for regenerative medicine, particularly for treatment of Parkinson's disease.

## Claims

1. A cell aggregate comprising FOXA2-positive or TUJ1-positive neural cells and comprising 1000 or more cells.

2. The cell aggregate according to claim 1, comprising about 70% or more of the FOXA2-positive or TUJ1-positive neural cells, based on a total number of cells.

3. The cell aggregate according to claim 1 or 2, wherein cell death can be suppressed during culture.

4. The cell aggregate according to any one of claims 1 to 3, further having at least one characteristic selected from the following:
(a1) equivalent circle diameter is 100 µm to 2000 µm;
(a2) convexity or solidity is 0.5 or more;
(a3) Feret diameter ratio is 0.5 or more; and
(a4) circularity is 0.3 or more.

5. The cell aggregate according to any one of claims 1 to 4, wherein the cell aggregate has no debris layer on a surface thereof, and a borderline of the cell aggregate is clear under a microscope.

6. A mixture of a plurality of cell aggregates, comprising 50% or more of the cell aggregate according to any one of claims 1 to 5, based on a total number of cell aggregates.

7. The mixture of cell aggregates according to claim 6, wherein at least one index selected from the group consisting of a circularity, a minimum diameter, a maximum diameter, a vertical Feret diameter or a horizontal Feret diameter, a Feret diameter ratio, an equivalent circle diameter, a perimeter, an area, and a convexity or a solidity has a coefficient of variation of 15% or less.

8. A method for producing a mixture of adherent cell populations, comprising steps of:
(1) inducing differentiation of a plurality of stem cells in the presence of a first differentiation-inducing factor to obtain a plurality of cells comprising one or more neuronal precursor cells in a first differentiation stage;
(2) selectively separating the neuronal precursor cells in a first differentiation stage from the plurality of cells obtained in step (1), wherein the separating step comprises
suspending the plurality of cells obtained in step (1) in a continuous flow of a liquid vehicle, and
distinguishing the neuronal precursor cells in a first differentiation stage, and separating the neuronal precursor cells in a first differentiation stage and other cells so as to let the neuronal precursor cells in a first differentiation stage and the other cells flow into different continuous flows of the liquid vehicle; and
(3) culturing the neuronal precursor cells in a first differentiation stage separated in step (2) in the presence of a second differentiation-inducing factor to obtain a mixture of adherent cell populations, wherein the mixture of adherent cell populations comprises 50% or more of adherent cell populations having the following characteristics (b1) and (b2), based on a total number of the adherent cell populations:
(b1) comprising neural cells in a second differentiation stage; and
(b2) comprising 1000 or more cells.

9. The production method according to claim 8, wherein cell death of the adherent cell populations having characteristics (b1) and (b2) can be suppressed during culture.

10. The production method according to claim 9, wherein when the adherent cell populations are cultured for 14 to 20 days, a number of cells at the completion of culture is 5% or more of a number of cells at the beginning of culture.

11. The production method according to any one of claims 8 to 10, wherein the mixture of adherent cell populations is a mixture of cell aggregates.

12. The production method according to claim 11, wherein the adherent cell populations are cell aggregates, and the cell aggregates having characteristics (b1) and (b2) have an equivalent circle diameter of 100 µm to 2000 µm.

13. The production method according to claim 12, wherein the adherent cell populations having characteristics (b1) and (b2) are cell aggregates, which further have the following characteristics:
(b3) convexity or solidity is 0.5 or more;
(b4) Feret diameter ratio is 0.5 or more; and
(b5) circularity is 0.3 or more.

14. The production method according to any one of claims 11 to 13, wherein at least one index selected from the group consisting of a circularity, a minimum diameter, a maximum diameter, a vertical Feret diameter or a horizontal Feret diameter, a Feret diameter ratio, an equivalent circle diameter, a perimeter, an area, and a convexity or a solidity of the mixture of cell aggregates has a coefficient of variation of 15% or less.

15. The production method according to any one of claims 8 to 14, wherein in step (2), the neuronal precursor cells in a first differentiation stage are separated using a micro-channel system cell sorter.

16. The production method according to any one of claims 8 to 15, wherein in step (2), the neuronal precursor cells in a first differentiation stage are separated in a closed system.

17. The production method according to any one of claims 8 to 16, wherein the stem cells are pluripotent stem cells.

18. The production method according to any one of claims 8 to 17, wherein the neuronal precursor cells in a first differentiation stage are neuronal precursor cells committed to a midbrain floor plate.

19. The production method according to claim 18, wherein the neuronal precursor cells in a first differentiation stage are Corin-positive and/or Lrtm1-positive cells.

20. The production method according to any one of claims 8 to 19, wherein the neural cells in a second differentiation stage are neural cells positive for at least one marker selected from the group consisting of TUJ1, OTX2, FOXA2, LMX1A, LMX1B, EN1, Nurr1, PITX3, DAT, GIRK2 and TH.

21. The production method according to claim 20, wherein the neural cells in a second differentiation stage are FOXA2-positive and TUJ1-positive dopaminergic neuron progenitor cells.

22. A mixture of adherent cell populations obtained by the production method according to any one of claims 8 to 21.

23. A method for producing an adherent cell population, comprising
separating the adherent cell populations having characteristics (b1) and (b2) from the mixture of adherent cell populations obtained by the production method according to any one of claims 8 to 21.

24. An adherent cell population obtained by the production method according to claim 23.

25. A pharmaceutical composition for transplantation, comprising any one of the cell aggregate according to any one of claims 1 to 5; the mixture of cell aggregates according to claim 6 or 7; the mixture of adherent cell populations according to claim 22; and the adherent cell population according to claim 24.

26. A therapeutic agent for a disease in need of supplement of neural cells, comprising any one of the cell aggregate according to any one of claims 1 to 5; the mixture of cell aggregates according to claim 6 or 7; the mixture of adherent cell populations according to claim 22; and the adherent cell population according to claim 24.

27. A method for treating a disease in need of supplement of neural cells, comprising transplanting any one of the cell aggregate according to any one of claims 1 to 5; the mixture of cell aggregates according to claim 6 or 7; the mixture of adherent cell populations according to claim 22; and the adherent cell population according to claim 24, into a central nerve of a patient.
